(19) Europäisches Patentamt
European Patent Office
Office européen des brevets

(11) Veröffentlichungsnummer: **0 024 582**
**A1**

(12) # EUROPÄISCHE PATENTANMELDUNG

(21) Anmeldenummer: **80104589.9**

(22) Anmeldetag: **04.08.80**

(51) Int. Cl.³: **C 07 D 487/04**
A 61 K 31/55
//(C07D487/04, 239/00, 243/00)

(30) Priorität: **10.08.79 CH 7334/79**
**10.08.79 CH 7335/79**

(43) Veröffentlichungstag der Anmeldung:
**11.03.81 Patentblatt 81/10**

(84) Benannte Vertragsstaaten:
**AT BE CH DE FR GB IT LI LU NL SE**

(71) Anmelder: **Byk Gulden Lomberg Chemische Fabrik GmbH**
**Byk-Gulden-Strasse 2**
**D-7750 Konstanz(DE)**

(72) Erfinder: **Schaefer, Hartmann, Dr.**
**Zum Purren 27**
**D-7750 Konstanz 16(DE)**

(72) Erfinder: **Riedel, Richard, Dr.**
**Brüelstrasse 22**
**D-7750 Konstanz(DE)**

(72) Erfinder: **Klemm, Kurt, Dr.**
**Im Weinberg 2**
**D-7753 Allensbach(DE)**

(72) Erfinder: **Senn-Bilfinger, Jörg, Dr.**
**Dorfplatz 5**
**D-7753 Allensbach 3(DE)**

(72) Erfinder: **Eltze Manfrid, Dr.**
**Schützenstrasse 20**
**D-7750 Konstanz(DE)**

(54) **Benzodiazepinone, Verfahren zu ihrer Herstellung sowie sie enthaltende Arzneimittel.**

(57) Benzodiazepinone der allgemeinen Formel I

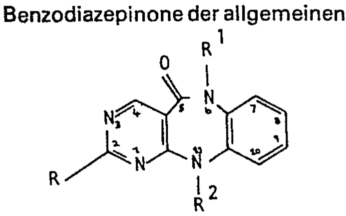

(I),

worin R ein Wasserstoffatom oder einen Phenylrest, $R^1$ ein Wasserstoffatom oder einen Alkylrest mit 1 bis 4 Kohlenstoffatomen, $R^2$ die Gruppe $-CO-C_nH_{2n}-R^3$ oder (wenn R ein Phenylrest ist) ein Wasserstoffatom, $R^3$ ein Halogenatom oder die Gruppe $-N(R^4)R^5$, $R^4$ einen Alkylrest mit 1 bis 4 Kohlenstoffatomen oder einen Alkenylrest mit 3 bis 5 Kohlenstoffatomen und $R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder $R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und n 1 oder 2 darstellt, sowie ihre N-Oxide und ihre Säureadditionssalze mit anorganischen und organischen Säuren sind neue Verbindungen. Sie werden in der pharmazeutischen Industrie als Zwischenprodukte und zur Herstellung von antiulcerogenen Medikamenten verwendet.

EP 0 024 582 A1

Croydon Printing Company Ltd.

Die Erfindung betrifft Benzodiazepinone, Verfahren zu ihrer Herstellung, ihre Verwendung sowie sie enthaltende Arzneimittel.

Die erfindungsgemäßen Verbindungen werden in der pharmazeutischen Industrie als Zwischenprodukte und zur Herstellung von Medikamenten verwendet.

In der deutschen Auslegeschrift DE-AS 17 95 183 werden Pyridobenzodiazepin-6-on-derivate mit ulkushemmender, sekretionshemmender, antitussiver und teilweise antiemetischer Wirkung beansprucht, während in der DE-AS 16 20 523 Pyridobenzodiazepin-5-one mit rein antitussiver Wirkung beschrieben werden. - Pyrimidobenzodiazepine sollen als Anti-Hypoxie-Mittel mit hypothermaler, antipyretischer und anti-inflammatorischer Wirkung Verwendung finden (DE-AS 24 18 285). - Pyrimido[4,5-b]-[1,5]benzodiazepin-5-on wird beschrieben, jedoch werden keine pharmakologischen Eigenschaften dieser Verbindung offenbart [K.J.M.Andrews, B.P.Tong, J.Chem.Soc.1753 (1968)].

Es wurde nun eine Klasse neuer Benzodiazepinone synthetisiert, die weder in den genannten Publikationen erwähnt noch durch sie nahegelegt wird. Diese Benzodiazepinone besitzen interessante und besonders vorteilhafte pharmakologische Eigenschaften.

Gegenstand der Erfindung sind Benzodiazepinone der allgemeinen Formel I

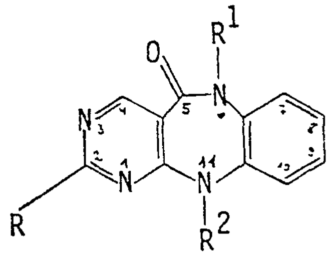

(I),

worin

R ein Wasserstoffatom oder einen Phenylrest,

$R^1$ ein Wasserstoffatom oder einen Alkylrest mit 1 bis 4 Kohlenstoffatomen,

$R^2$ die Gruppe $-CO-C_nH_{2n}-R^3$ oder (wenn R ein Phenylrest ist) ein Wasser-

stoffatom,

$R^3$ ein Halogenatom oder die Gruppe $-N(R^4)R^5$,

$R^4$ einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihre N-Oxide und ihre Säureadditionssalze mit anorganischen und organischen Säuren.

Alkylreste mit 1 bis 4 Kohlenstoffatomen sind der Methyl-, Ethyl-, Propyl-, Butyl-, Isopropyl-, Isobutyl-, sek.-Butyl und tert.-Butylrest.

Als Alkenylreste mit 3 bis 5 Kohlenstoffatomen seien der Allylrest und der 2-Methylallylrest genannt.

Als Halogenatome seien das Chlor-, Brom- und Jodatom, bevorzugt das Chlor- und Bromatom, insbesondere das Chloratom genannt.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat (2-(4-Hydroxybenzoyl)-benzoat), Fendizoat (o-[(2'-Hydroxy-4-biphenylyl)-carbonyl]-benzoat), Propionat, Butyrat, Sulfosalicylat, Maleat, Laurat, Malat, Fumarat, Succinat, Oxalat, Tartrat, Amsonat (4,4'-Diamino-stilben-2,2'-disulfonat), Embonat (4,4'-Methylen-bis-3-hydroxy-2-naphthoat), Metembonat (4,4'-Methylen-bis-3-methoxy-2-naphthoat), Stearat, Tosilat (p-Toluolsulfonat), 2-Hydroxy-3-naphthoat, 3-Hydroxy-2-naphthoat, Mesilat (Methansulfonat).

Eine Ausgestaltung der Erfindung sind Benzodiazepinone der allgemeinen Formel $I^a$

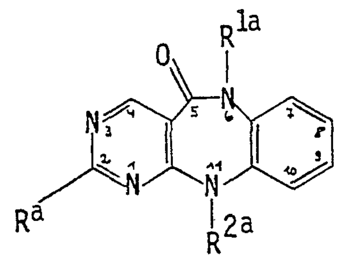

$(I^a)$,

worin

$R^a$ einen Phenylrest,

$R^{1a}$ ein Wasserstoffatom oder einen Alkylrest mit 1 bis 4 Kohlenstoffatomen,

$R^{2a}$ die Gruppe $-CO-C_{n^a}H_{2n^a}-R^{3a}$,

$R^{3a}$ die Gruppe $-N(R^{4a})R^{5a}$,

$R^{4a}$ einen Alkylrest mit 1 bis 4 Kohlenstoffatomen oder einen Alkenylrest mit 3 bis 5 Kohlenstoffatomen und

$R^{5a}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4a}$ und $R^{5a}$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihre N-Oxide und ihre Säureadditionssalze mit anorganischen und organischen Säuren.

Besonders erwähnenswerte Vertreter der Ausgestaltung $I^a$ sind solche, in denen $R^a$, $R^{1a}$, $R^{2a}$ und $R^{3a}$ die oben angegebenen Bedeutungen haben, $R^{4a}$ einen Alkylrest mit 1 bis 4 Kohlenstoffatomen oder einen Alkenylrest mit 3 Kohlenstoffatomen darstellt, $R^{5a}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4a}$ und $R^{5a}$ gemeinsam eine Pyrrolidino-, Piperidino-, Morpholino- oder 4-Methyl- oder 4-Ethyl-1-piperazinylgruppe darstellen, und $n^a$ die Bedeutung 1 hat, und ihre Säureadditionssalze.

Bevorzugte Vertreter der Ausgestaltung $I^a$ sind solche, in denen $R^a$, $R^{2a}$ und $R^{3a}$ die oben angegebenen Bedeutungen haben, $R^{1a}$ einen Methyl-, n-Propyl-, Isopropyl-, n-Butyl- oder sec.-Butylrest darstellt, $R^{4a}$ einen Methylrest darstellt, $R^{5a}$ einen Methyl- oder Ethylrest darstellt, der endständig durch eine Dimethyl- oder Diethylaminogruppe substituiert ist, oder $R^{4a}$ und $R^{5a}$ gemeinsam eine Pyrrolidino-, Piperidino- oder 4-Methyl-1-piperazinylgruppe darstellen, und $n^a$ die Bedeutung 1 hat, und ihre pharmakologisch verträglichen Säureadditionssalze.

Besonders bevorzugte Vertreter der Ausgestaltung $I^a$ sind solche, in denen $R^a$, $R^{2a}$ und $R^{3a}$ die oben angegebenen Bedeutungen haben, $R^{1a}$ einen Methylrest darstellt, $R^{4a}$ einen Methylrest und $R^{5a}$ einen 2-Dimethylaminoethylrest darstellt, oder $R^{4a}$ und $R^{5a}$ gemeinsam eine Piperidino- oder 4-Methyl-1-piperazinylgruppe darstellen, und $n^a$ die Bedeutung 1 hat, und ihre pharmakologisch verträglichen Säureadditionssalze.

Eine weitere Ausgestaltung der Erfindung sind Benzodiazepinone der allgemeinen Formel $I^b$

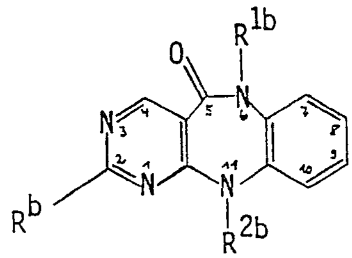

($I^b$),

worin

$R^b$ einen Phenylrest,

$R^{1b}$ ein Wasserstoffatom oder einen Alkylrest mit 1 bis 4 Kohlenstoffatomen,

$R^{2b}$ ein Wasserstoffatom oder die Gruppe $-CO-C_{n^b}H_{2n^b}-R^{3b}$,

$R^{3b}$ ein Halogenatom und

$n^b$ 1 oder 2 darstellt,

sowie ihre N-Oxide und ihre Säureadditionssalze mit anorganischen und organischen Säuren.

Bevorzugte Vertreter der Ausgestaltung $I^b$ sind solche, in denen $R^b$ und $R^{2b}$ die oben angegebenen Bedeutungen haben, $R^{1b}$ einen Methyl-, n-Propyl-, Isopropyl-, n-Butyl- oder sec.-Butylrest darstellt, $R^{3b}$ Chlor oder Brom bedeutet und $n^b$ 1 darstellt und ihre Säureadditionssalze.

Besonders bevorzugte Vertreter der Ausgestaltung $I^b$ sind solche, in denen $R^b$ und $R^{2b}$ die oben angegebenen Bedeutungen haben, $R^{1b}$ einen Methylrest darstellt, $R^{3b}$ Chlor bedeutet und $n^b$ 1 darstellt, und ihre Säureadditionssalze.

Eine weitere Ausgestaltung der Erfindung sind Benzodiazepinone der allgemeinen Formel $I^c$

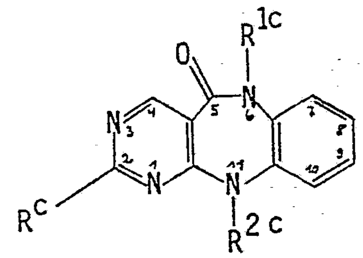

$(I^c)$,

worin

$R^c$ ein Wasserstoffatom,

$R^{1c}$ ein Wasserstoffatom oder einen Alkylrest mit 1 bis 4 Kohlenstoffatomen,

$R^{2c}$ die Gruppe $-CO-C_ncH_{2n}c-R^{3c}$,

$R^{3c}$ die Gruppe $-N(R^{4c})R^{5c}$ ,

$R^{4c}$ einen Alkylrest mit 1 bis 4 Kohlenstoffatomen oder einen Alkenylrest mit 3 bis 5 Kohlenstoffatomen und

$R^{5c}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4c}$ und $R^{5c}$ gemeinsam unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

$n^c$ 1 oder 2 darstellt,

sowie ihre N-Oxide und ihre Säureadditionssalze mit anorganischen und organischen Säuren.

Besonders erwähnenswerte Vertreter der Ausgestaltung $I^c$ sind solche, in denen $R^c$, $R^{1c}$, $R^{2c}$ und $R^{3c}$ die oben angegebenen Bedeutungen haben, $R^{4c}$ einen Alkylrest mit 1 bis 4 Kohlenstoffatomen oder einen Alkenylrest mit 3 Kohlenstoffatomen darstellt, $R^{5c}$ einen Alkylrest mit 1 bis 4 Kohlenstoffatomen, der gegebenenfalls endständig durch eine Dimethylamino- oder Diethylaminogruppe substituiert ist, oder einen

Alkenylrest mit 3 Kohlenstoffatomen darstellt, oder $R^{4c}$ und $R^{5c}$ gemeinsam eine Pyrrolidino-, Piperidino-, Morpholino- oder 4-Methyl- oder 4-Ethyl-1-piperazinylgruppe darstellen, und $n^c$ die Bedeutung 1 hat, und ihre Säureadditionssalze.

Bevorzugte Vertreter der Ausgestaltung $I^c$ sind solche, in denen $R^c$, $R^{2c}$ und $R^{3c}$ die oben angegebenen Bedeutungen haben, $R^{1c}$ ein Wasserstoffatom oder eine Methyl- oder n-Butylgruppe darstellt, $R^{4c}$ einen Methylrest darstellt, $R^{5c}$ einen Methyl- oder Ethylrest darstellt, der endständig durch eine Dimethyl- oder Diethylaminogruppe substituiert ist, oder $R^{4c}$ und $R^{5c}$ gemeinsam eine Pyrrolidino-, Piperidino-, Morpholino- oder 4-Methyl-1-piperazinylgruppe darstellen, und $n^c$ die Bedeutung 1 hat, und ihre pharmakologisch verträglichen Säureadditionssalze.

Besonders bevorzugte Vertreter der Ausgestaltung $I^c$ sind solche, in denen $R^c$, $R^{2c}$ und $R^{3c}$ die oben angegebenen Bedeutungen haben, $R^{1c}$ ein Wasserstoffatom oder eine Methylgruppe darstellt, $R^{4c}$ und $R^{5c}$ gemeinsam eine Piperidino- oder 4-Methyl-1-piperazinylgruppe darstellen und $n^c$ die Bedeutung 1 hat, und ihre pharmakologisch verträglichen Säureadditionssalze.

Eine weitere Ausgestaltung der Erfindung sind Benzodiazepinone der allgemeinen Formel $I^d$

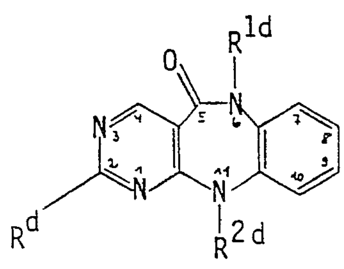

($I^d$),

worin

$R^d$ ein Wasserstoffatom,

$R^{1d}$ ein Wasserstoffatom oder einen Alkylrest mit 1 bis 4 Kohlenstoffatomen,

$R^{2d}$ die Gruppe $-CO-C_{n^d}H_{2n^d}-R^{3d}$,

$R^{3d}$ ein Halogenatom und

$n^d$ 1 oder 2 darstellt,
sowie ihre N-Oxide und ihre Säureadditionssalze mit anorganischen und organischen Säuren.

Bevorzugte Vertreter der Ausgestaltung $I^d$ sind solche, in denen $R^d$ und $R^{2d}$ die oben genannten Bedeutungen haben, $R^{1d}$ ein Wasserstoffatom oder eine Methyl- oder n-Butylgruppe darstellt, $R^{3d}$ ein Chlor- oder Bromatom bedeutet und $n^d$ 1 darstellt, und ihre Säureadditionssalze.

Besonders bevorzugte Vertreter der Ausgestaltung $I^d$ sind solche, in denen $R^d$ und $R^{2d}$ die oben genannten Bedeutungen haben, $R^{1d}$ ein Wasserstoffatom oder eine Methylgruppe darstellt, $R^{3d}$ ein Chloratom bedeutet und $n^d$ 1 darstellt, und ihre Säureadditionssalze.

Als Vertreter erfindungsgemäßer Verbindungen seien beispielsweise genannt:
6-Isopropyl-11-[(4-methyl-1-piperazinyl)-acetyl]-2-phenyl-5.6-dihydropyrimido[4.5-b][1.5]-benzodiazepinon-(5),
6-Butyl-11-diallylaminoacetyl-2-phenyl-5.6-dihydropyrimido[4.5-b][1.5]-benzodiazepinon-(5),
6-Methyl-11-[2-(dimethylamino)-propionyl]-2-phenyl-5.6-dihydropyrimido-[4.5-b][1.5]-benzodiazepinon-(5),
6-Methyl-11-pyrrolidino-acetyl-2-phenyl-5.6-dihydropyrimido[4.5-b]-[1.5]-benzodiazepinon-(5),
6-Ethyl-11-diisopropylaminoacetyl-2-phenyl-5.6-dihydropyrimido[4.5-b]-[1.5]-benzodiazepinon-(5),
6-Propyl-11-[(4-ethyl-1-piperazinyl)-acetyl]-2-phenyl-5.6-dihydropyrimido[4.5-b][1.5]-benzodiazepinon-(5),
6-Methyl-11-perhydroazepino-acetyl-2-phenyl-5.6-dihydropyrimido[4.5-b]-[1.5]-benzodiazepinon-(5),
6-Isopropyl-11-piperidino-acetyl-2-phenyl-5.6-dihydropyrimido[4.5-b]-[1.5]-benzodiazepinon-(5),
6-Methyl-11-[3-(piperidino)-propionyl]-2-phenyl-5.6-dihydropyrimido-[4.5-b][1.5]-benzodiazepinon-(5),

6-Ethyl-11-(n-butyl-tert.-butylamino)-acetyl-2-phenyl-5.6-dihydropyrimido[4.5-b][1.5]-benzodiazepinon-(5),
6-Butyl-11-dibutylaminoacetyl-2-phenyl-5.6-dihydropyrimido[4.5-b]-[1.5]-benzodiazepinon-(5),
6-Methyl-11-[2-(dibutylamino)-propionyl]-2-phenyl-5.6-dihydropyrimido-[4.5-b][1.5]-benzodiazepinon-(5),
11-[(4-Methyl-1-piperazinyl)-acetyl]-2-phenyl-5.6-dihydropyrimido[4.5-b][1.5]-benzodiazepinon-(5),
6-iso-Butyl-11-[(4-methyl-1-piperazinyl)-acetyl]-2-phenyl-5.6-dihydropyrimido[4.5-b][1.5]-benzodiazepinon-(5),
11-Piperidino-acetyl-2-phenyl-5.6-dihydropyrimido[4.5-b]-[1.5]benzodiazepinon-(5),
6-sec.-Butyl-11-[(4-methyl-1-piperazinyl)-acetyl]-2-phenyl-5.6-dihydropyrimido[4.5-b][1.5]-benzodiazepinon-(5),
6-Ethyl-11-[2-(diallylamino)-propionyl]-2-phenyl-5.6-dihydropyrimido-[4.5-b][1.5]-benzodiazepinon-(5),
6-Methyl-11-dimethylaminoacetyl-2-phenyl-5.6-dihydropyrimido[4.5-b]-[1.5]-benzodiazepinon-(5),
6-Isopropyl-11-perhydroazepino-acetyl-2-phenyl-5.6-dihydropyrimido-[4.5-b][1.5]benzodiazepinon-(5),
6-Methyl-11-[3-(diisopropylamino)-propionyl]-5.6-dihydropyrimido-[4.5-b][1.5]benzodiazepinon-(5),
6-Ethyl-11-(n-butyl-tert.-butylamino)-acetyl-5.6-dihydropyrimido-[4.5-b][1.5]benzodiazepinon-(5),
6-n-Butyl-11-di-n-butylaminoacetyl-5.6-dihydropyrimido[4.5-b]-[1.5]benzodiazepinon-(5),
6-Methyl-11-[2-(di-n-butylamino)-propionyl]-5.6-dihydropyrimido-[4.5-b][1.5]benzodiazepinon-(5),

6-Methyl-11-[(4-methyl-1-piperazinyl)-acetyl]-5.6-dihydropyrimido-[4.5-b][1.5]benzodiazepinon-(5),
11-[2-(4-Methyl-1-piperazinyl)-propionyl]-5.6-dihydropyrimido-[4.5-b][1.5]benzodiazepinon-(5),
6-Methyl-11-[3-(4-methyl-1-piperazinyl)-propionyl]-5.6-dihydropyrimido-[4.5-b][1.5]benzodiazepinon-(5),
6-sec.-Butyl-11-[(4-methyl-1-piperazinyl]-acetyl]-5.6-dihydropyrimido-[4.5-b][1.5]benzodiazepinon-(5),
6-Ethyl-11-[3-(diallylamino)-propionyl]-5.6-dihydropyrimido-[4.5-b][1.5]benzodiazepinon-(5),
6-Methyl-11-dimethylaminoacetyl-5.6-dihydropyrimido[4.5-b][1.5]-benzodiazepinon-(5),
11-Perhydroazepino-acetyl-5.6-dihydropyrimido[4.5-b][1.5]benzodiazepinon-(5),
11-Diallylaminoacetyl-5.6-dihydropyrimido[4.5-b][1.5]benzodiazepinon-(5),
6-Methyl-11-pyrrolidino-acetyl-5.6-dihydropyrimido[4.5-b][1.5]-benzodiazepinon-(5),
6-Ethyl-11-diisopropylaminoacetyl-5.6-dihydropyrimido[4.5-b]-[1.5]benzodiazepinon-(5),
11-[(4-Ethyl-1-piperazinyl)-acetyl]-5.6-dihydropyrimido[4.5-b]-[1.5]benzodiazepinon-(5),
6-Methyl-11-perhydroazepino-acetyl-5.6-dihydropyrimido[4.5-b]-[1.5]benzodiazepinon-(5),
6-Isopropyl-11-piperidino-acetyl-5.6-dihydropyrimido[4.5-b][1.5]-benzodiazepinon-(5)
und ihre N-Oxide und ihre Säureadditionssalze,
insbesondere
11-[(4-Methyl-1-piperazinyl)-acetyl]-5.6-dihydropyrimido[4.5-b][1.5]-benzodiazepinon-(5),
6-Methyl-2-phenyl-11-piperidinoacetyl-5.6-dihydropyrimido[4.5-b][1.5]-benzodiazepinon-(5),
6-n-Butyl-11-[(4-methyl-1-piperazinyl)-acetyl]-5.6-dihydropyrimido-[4.5-b][1.5]benzodiazepinon-(5),
6-Methyl-11-piperidinoacetyl-5.6-dihydropyrimido[4.5-b][1.5]benzodiazepinon-(5),

6-Methyl-2-phenyl-11-pyrrolidinoacetyl-5.6-dihydropyrimido[4.5-b]-[1.5]benzodiazepinon-(5),
6-Isopropyl-2-phenyl-11-[(4-methyl-1-piperazinyl)-acetyl]-5.6-dihydropyrimido[4.5-b][1.5]benzodiazepinon-(5),
6-sec.-Butyl-2-phenyl-11-[(4-methyl-1-piperazinyl)-acetyl]-5.6-dihydropyrimido[4.5-b][1.5]benzodiazepinon-(5),
11-[N-(2-dimethylaminoethyl)-N-methylamino]-acetyl-6-methyl-2-phenyl-5.6-dihydropyrimido[4.5-b][1.5]benzodiazepinon-(5),
6-Methyl-11-[(4-methyl-1-piperazinyl)-acetyl]-2-phenyl-5.6-dihydropyrimido[4.5-b][1.5]benzodiazepinon-(5),
2-Phenyl-11-piperidinoacetyl-5.6-dihydropyrimido[4.5-b][1.5]benzodiazepinon-(5)
und ihre Säureadditionssalze.

Die Benzodiazepinone der allgemeinen Formel I bzw. die der Ausgestaltungen $I^a$, $I^b$, $I^c$ und $I^d$ und ihre N-Oxide und ihre Säureadditionssalze besitzen wertvolle Eigenschaften, die sie gewerblich verwertbar machen.

Die Benzodiazepinone der allgemeinen Formel I, worin $R^2$ die Gruppe $-CO-C_nH_{2n}-R^3$ und $R^3$ die Gruppe $-N(R^4)R^5$ darstellt, und worin R, $R^1$, $R^4$, $R^5$ und n die oben angegebenen Bedeutungen haben, sowie die Benzodiazepinone der allgemeinen Formeln $I^a$ und $I^c$, und N-Oxide und Säureadditionssalze dieser Verbindungen sind erfindungsgemäße pharmakologisch wirksame Verbindungen, die durch eine ausgezeichnete Magen- und Darmschutzwirkung bei Warmblütern gekennzeichnet sind. Sie hemmen insbesondere die Magen- und Darmgeschwürbildung.

Ferner weisen sie, bedingt durch eine geringe Toxizität und das Fehlen wesentlicher Nebenwirkungen, eine günstige therapeutische Breite auf. Im übrigen haben die Verbindungen nur eine geringe anticholinerge Wirkung, was sich beispielsweise an der geringen Hemmung der durch Carbachol ausgelösten Salivation feststellen läßt.

Die erfindungsgemäßen Benzodiazepinone der allgemeinen Formel I, worin $R^2$ ein Wasserstoffatom oder die Gruppe $-CO-C_nH_{2n}-R^3$ darstellt und $R^3$ ein Halogenatom ist, und die Benzodiazepinone der allgemeinen Formeln $I^b$ und $I^d$ sind wertvolle Zwischenprodukte für die Herstellung der pharmakologisch wirksamen Verbindungen.

Die ausgezeichnete Wirksamkeit der pharmakologisch wirksamen Benzodiazepinone und ihrer pharmakologisch verträglichen N-Oxide und entsprechenden Säureadditionssalze ermöglicht ihren Einsatz in der Human- oder Veterinärmedizin, wobei sie zur Behandlung und Prophylaxe von Krankheiten, die auf Erkrankungen des Magens oder Darms beruhen, verwendet werden. Beispielsweise werden akuter und chronischer Ulcus ventriculi und Ulcus duodeni, Gastritis, hyperacider Reizmagen und medikamentös bedingte Magenbeschwerden bei Mensch oder Tier behandelt.

Ein weiterer Gegenstand der Erfindung ist daher ein Verfahren zur Behandlung von Säugetieren, die an einer der obengenannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der oben genannten pharmakologisch wirksamen Verbindungen verabreicht.

Gegenstand der Erfindung ist außerdem die Verwendung der hier genannten erfindungsgemäßen Verbindungen bei der Bekämpfung der oben angegebenen Krankheiten.

Ebenso umfaßt die Erfindung die Verwendung der Verbindungen bei der Herstellung von Arzneimitteln, die zur Bekämpfung der angeführten Krankheiten eingesetzt werden.

Ein weiterer Gegenstand der Erfindung sind Arzneimittel, die ein oder mehrere Benzodiazepinone der allgemeinen Formel I*

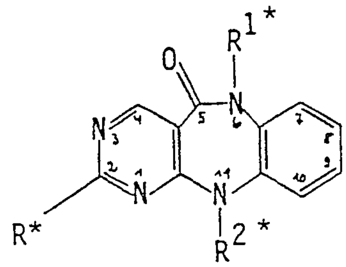

(I*),

worin

$R^*$ ein Wasserstoffatom oder einen Phenylrest,

$R^{1*}$ ein Wasserstoffatom oder einen Alkylrest mit 1 bis 4 Kohlenstoffatomen,

$R^{2*}$ die Gruppe $-CO-C_{n^*}H_{2n^*}-R^{3*}$,

$R^{3*}$ die Gruppe $-N(R^{4*})R^{5*}$,

$R^{4*}$ einen Alkylrest mit 1 bis 4 Kohlenstoffatomen oder einen Alkenylrest mit 3 bis 5 Kohlenstoffatomen und

$R^{5*}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^{4*}$ und $R^{5*}$ gemeinsam und unter Einschluß des Stickstoffatoms an das sie gebunden sind einen Pyrrolidino-, Piperidino-, Morpholino-, Perhydropazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

$n^*$ 1 oder 2 darstellt,

und/oder ihre pharmakologisch verträglichen N-Oxide und/oder ihre pharmakologisch verträglichen Säureadditionssalze mit anorganischen und organischen Säuren enthalten.

Ausgestaltungen der Arzneimittel sind solche, die erfindungsgemäße Benzodiazepinone der allgemeinen Formeln $I^a$ oder $I^c$ und/oder ihre pharmakologisch verträglichen N-Oxide und/oder entsprechende Säureadditionssalze enthalten.

Die Arzneimittel werden nach an sich bekannten Verfahren hergestellt, wobei die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 0,5 bis 95, vorzugsweise 15 bis 75 Gewichtsprozent der Gesamtmischung.

In Übereinstimmung mit der Erfindung werden die Wirkstoffe in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale, rektale oder parenterale Gabe in geeigneten Dosen erreicht werden. Üblicherweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 1 bis 300 mg vorteilhafterweise 5 bis 100 mg und insbesondere 10 bis 50 mg Wirkstoff, enthalten.

Im allgemeinen hat es sich in der Humanmedizin als vorteilhaft erwiesen, den oder die Wirkstoffe bei oraler Gabe in einer Tagesdosis von 0,01 bis 12, vorzugsweise 0,07 bis 4, insbesondere 0,15 bis 2 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von 0,01 bis 4, vorzugsweise 0,07 bis 1,4, insbesondere 0,15 bis 0,7 mg/kg Körpergewicht. Bei einer parenteralen, z.B. intravenösen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genanntenDosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs,

eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Zur oralen Anwendung können z.B. Tabletten, Dragees, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Die orale Anwendung der Arzneistoffe ist bevorzugt.

Der oder die Wirkstoffe können gegebenenfalls mit einem oder mehreren der angegebenen Träger- oder Zusatzstoffe auch in mikroverkapselter Form formuliert werden.

Sollen die erfindungsgemäßen, pharmakologisch wirksamen Verbindungen und/oder ihre N-Oxide und/oder ihre Säureadditionssalze zur Behandlung der oben genannten Krankheiten eingesetzt werden, so können die pharmazeutischen Zubereitungen auch einen oder mehrere andere pharmakologisch aktive Bestandteile anderer Arzneimittelgruppen, wie

Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine, Aminosäuren etc. enthalten.

Ein weiterer Gegenstand der Erfindung ist ein Verfahren zur Herstellung von Benzodiazepinone der allgemeinen Formel I,

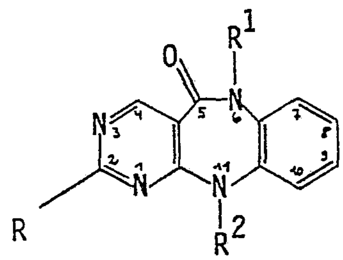

(I),

worin

R ein Wasserstoffatom oder einen Phenylrest,

$R^1$ ein Wasserstoffatom oder einen Alkylrest mit 1 bis 4 Kohlenstoffatomen,

$R^2$ die Gruppe $-CO-C_nH_{2n}-R^3$,

$R^3$ ein Halogenatom oder die Gruppe $-N(R^4)R^5$,

$R^4$ einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihrer N-Oxide und ihrer Säureadditionssalze mit anorganischen und organischen Säuren, das dadurch gekennzeichnet ist, daß man

Benzodiazepinone der allgemeinen Formel II

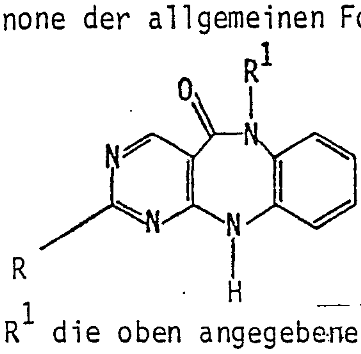

(II),

worin R und $R^1$ die oben angegebenen Bedeutungen haben, mit Verbindungen der allgemeinen Formel III

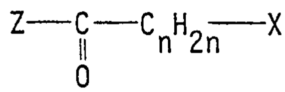

(III),

worin n die oben angegebene Bedeutung hat, X ein Halogenatom (Chlor, Brom, Iod) und Z eine Fluchtgruppe darstellt, umsetzt und gegebenenfalls anschließend mit Aminen der allgemeinen Formel IV

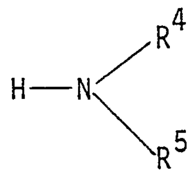

(IV),

worin $R^4$ und $R^5$ die oben angegebenen Bedeutungen haben, reagieren läßt und erhaltene Produkte gewünschtenfalls in die N-Oxide und/oder in die Säureadditionssalze überführt.

Die Umsetzung der Benzodiazepinone II mit den Verbindungen III erfolgt in an sich bekannter Weise.

Die Fluchtgruppe Z kann beispielsweise ein Halogenatom, insbesondere ein Chloratom, oder die Gruppe $X-C_nH_{2n}-CO-O-$ sein. Wenn Z ein Halogenatom ist, so wird die Reaktion vorteilhafterweise in Gegenwart eines säurebindenden Mittels (Protonenakzeptors) durchgeführt. Als geeignete Protonenakzeptoren seien beispielsweise genannt Alkalimetallcarbonate oder -bicarbonate, wie Natriumcarbonat oder Kaliumhydrogencarbonat; oder tertiäre organische Amine, wie Pyridin, Triethylamin oder Ethyldiisopropylamin.

Die Reaktion wird bevorzugt in inerten, wasserfreien Lösungsmitteln durchgeführt. Beispielsweise seien genannt: chlorierte Kohlenwasserstoffe, wie Methylenchlorid oder Chloroform; offenkettige oder cyclische Ether, wie Diethylether, Diethylenglycoldimethylether, Tetrahydrofuran oder insbesondere Dioxan; oder aromatische Kohlenwasserstoffe, wie Benzol oder Toluol.

Die Reaktionstemperatur, die von der Art der Fluchtgruppe, des Protonenakzeptors, der Reaktanten und des Lösungsmittels abhängig ist, liegt im allgemeinen zwischen 20 und 150°C, insbesondere zwischen 60 und 110°C, wobei die jeweilige Siedetemperatur des verwendeten Lösungsmittels bevorzugt ist. Die Reaktionszeit schwankt zwischen 20 Minuten und 60 Stunden.

Die Reaktion von II mit III führt zu Benzodiazepinonen I, worin $R^3$ ein Halogenatom ist ($R^3$ = X). Die Ausgestaltungen der Formeln $I^b$ und $I^d$ werden durch analoge Reaktionen der Verbindungen $II^b$ bzw. $II^d$ mit Verbindungen $III^b$ bzw. $III^d$, in denen die entsprechenden Substituenten $R^b$, $R^d$, $R^{1b}$, $R^{1d}$, $n^b$ und $n^d$ die oben angegebenen Bedeutungen haben, erhalten. Die so erhaltenen Verbindungen I (mit $R^3$ = X), $I^b$ bzw. $I^d$ sind wertvolle Zwischenprodukte.

Die gegebenenfalls sich anschließende Aminierung mit den Verbindungen der Formel IV, $IV^a$ bzw. $IV^c$, worin die entsprechenden Substituenten $R^4$, $R^5$, $R^{4a}$, $R^{5a}$, $R^{4c}$ und $R^{5c}$ die oben angegebenen Bedeutungen haben, führt zu Benzodiazepinonen I, in denen $R^3$ die Bedeutung $-N(R^4)R^5$ hat, bzw. zu den Benzodiazepinonen $I^a$ und $I^c$.

Die Reaktion der Zwischenprodukte mit den Aminen IV wird in an sich bekannter Weise und vorteilhafterweise in der Gegenwart eines Protonenakzeptors durchgeführt. Als solche eignen sich beispielsweise Alkalimetallcarbonate, wie Natriumcarbonat,oder Kaliumcarbonat, oder tertiäre Amine, wie Pyridin, Triethylamin oder Ethyldiisopropylamin.

Um Nebenreaktionen zu vermeiden kann vorteilhafterweise auch ein Oberschuß an Verbindung IV als Protonenakzeptor eingesetzt werden. In diesem Fall wird die Reaktion mit einem beispielsweise 2- bis 5-fachen Oberschuß an Verbindung IV durchgeführt.

Die Reaktion wird in geeigneten, vorteilhafterweise inerten, wasserfreien Lösungsmitteln, wie niederen Alkoholen (z.B. Methanol, Ethanol oder Isopropanol) offenkettigen oder cyclischen Ethern (z.B. Tetrahydrofuran oder insbeondere Dioxan), aromatischen Kohlenwasserstoffen (z.B. Benzol oder insbesondere Toluol), oder chlorierten Kohlenwasserstoffen (z.B. Methylenchlorid) durchgeführt.

Die Reaktionstemperatur kann zwischen $0^{0}$ und $150^{0}C$ liegen, wobei Temperaturen zwischen $50^{0}$ und $110^{0}C$ - insbesondere die Siedetemperatur des verwendeten Lösungsmittels - bevorzugt sind. Die Reaktionszeit liegt - in Abhängigkeit vom eingesetzten Amin - zwischen einigen Minuten und mehreren Stunden; gegebenenfalls kann die Reaktion durch Zugabe eines Alkalimetalliodides beschleunigt werden.

Bei Verwendung leichtflüchtiger Amine wird die Reaktion bevorzugt bei niedrigeren Temperaturen oder in einer geschlossenen Apparatur durchgeführt.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßrigem Natriumhydrogencarbonat, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

N-Oxide erhält man durch Oxidation der Benzodiazepinone mit geeigneten Oxidationsmitteln, wie Peroxidverbindungen, z.B. Wasserstoffperoxid oder m-Chlorperoxibenzoesäure, in geeigneten inerten Lösungsmitteln, z.B. Dichlormethan, in dem Fachmann bekannter Weise (siehe auch Houben-Weyl 11/2, 190-205).

Zu Verbindungen der Formel II gelangt man auf verschiedenen Wegen. So werden Verbindungen II, in denen $R^1$ einen Alkylrest bedeutet, durch Alkylierung der entsprechenden Verbindung II, in denen $R^1$ ein Wasserstoffatom bedeutet, in an sich bekannter Weise erhalten. Zur Deprotonierung der Verbindung II, in der $R^1$ ein Wasserstoffatom bedeutet, werden geeignete Basen, z.B. Natriumhydrid, Natriumamid oder Kalium-t-butanolat, verwendet. Die Deprotonierung wird in inerten, wasserfreien, gegebenenfalls polaren Lösungsmitteln, wie Dimethylsulfoxid oder Dioxan, durchgeführt. Die anschließende Alkylierung, beispielsweise mit Dialkylsulfaten, wie Dimethylsulfat, oder Alkylhalogeniden, wie Butyliodid, führt zu den gewünschten Produkten II, in denen $R^1$ einen Alkylrest darstellt.

Zu Produkten II, in denen $R^1$ einen Alkylrest darstellt, aber auch zu Produkten II, in denen $R^1$ ein Wasserstoffatom darstellt, gelangt man auch durch Cyclisierung von Verbindungen der allgemeinen Formel V

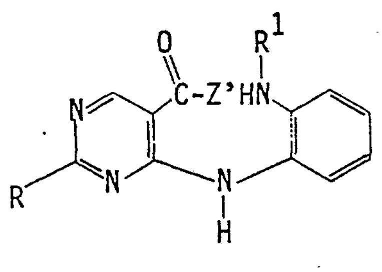

(V),

worin R und $R^1$ die oben angegebene Bedeutung haben und Z' eine geeignete Fluchtgruppe darstellt.

Eine geeignete Fluchtgruppe Z' ist eine Gruppe, die zusammen mit der Carbonylgruppe, an die sie gebunden ist, ein reaktives Carbonsäurederivat bildet.

Eine geeignete Fluchtgruppe Z' ist beispielsweise eine Alkoxygruppe, oder eine Hydroxygruppe, oder insbesondere eine OH-Gruppe, die in situ durch Reaktion mit Tetrachlorkohlenstoff/Triphenylphosphin in ein Chloratom umgewandelt wird [siehe L.C.Barstow et.al., J.Org.Chem. 35, 1305(1971)].

Die Cyclisierung wird - je nach Art der Fluchtgruppe - in an sich bekannter Weise durchgeführt:

Ist Z' eine Alkoxygruppe, so wird sie in inerten, bevorzugt polaren organischen Lösungsmitteln, wie niederen Alkoholen, z.B. Ethanol, gegebenenfalls in Gegenwart einer Säure, wie Halogenwasserstoffsäure, oder bevorzugt in Gegenwart einer Base, z.B. eines Alkalimetallalkanolats, bei Temperaturen zwischen $50^0$ und $100^0C$, bevorzugt bei der Siedetemperatur des verwendeten Lösungsmittels durchgeführt.

Ist Z' eine Hydroxygruppe, so wird der Ringschluß in inerten Lösungsmitteln und bevorzugt in Gegenwart eines Kondensationsmittels, wie Dicyclohexylcarbodiimid, durchgeführt.

Wenn Z' eine OH-Gruppe ist, die in situ in ein Chloratom umgewandelt wird, so wird die Cyclisierung in inerten Lösungsmitteln, z.B. in Dioxan oder Tetrahydrofuran, bei Temperaturen zwischen $50^0$ und $100^0C$, bevorzugt bei der Siedetemperatur des verwendeten Lösungsmittels, durchgeführt.

Verbindungen der Formel V werden durch Reaktion von Pyrimidinen VI mit o-Phenylendiaminen VII

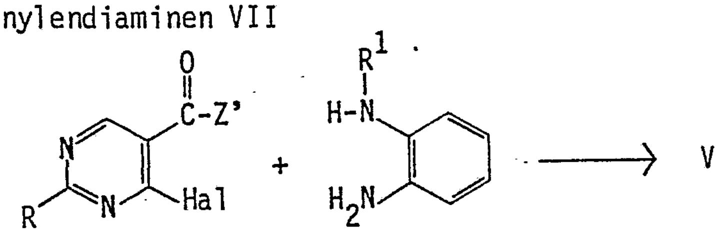

(VI) (VII)

worin R, $R^1$ und Z' die oben angegebenen Bedeutungen haben und Hal ein Halogenatom, insbesondere ein Chloratom darstellt, in an sich bekannter Weise hergestellt.

Die Umsetzung erfolgt beispielsweise in inerten, polaren organischen Lösungsmitteln, wie Ethanol oder Ethylacetat, unter Zusatz eines Protonenakzeptors, wie Triethylamin oder Natriumcarbonat, bei der Siedetemperatur des Lösungsmittels. Die als Ausgangsmaterial verwendeten Verbindungen der Formeln VI und VII sind entweder bekannt oder können nach bekannten Verfahren hergestellt werden.

Die folgenden Beispiele erläutern die Erfindung näher, ohne sie einzuschränken. Die Abkürzung Schmp. bedeutet Schmelzpunkt, Z. bedeutet Zersetzungspunkt. Unter "Ether" wird Diethylether verstanden.

Beispiele

Beispiel 1

11-[(4-Methyl-1-piperazinyl)-acetyl]-5,6-dihydropyrimido[4.5-b][1.5]-benzodiazepinon-(5)

Zu einer Suspension von 2,89 g (0,01 mol) 11-Chloracetyl-5,6-dihydro-pyrimido[4.5-b][1.5]benzodiazepinon-(5) in 30 ml absolutem Benzol gibt man 2,00 g (0.02 mol) N-Methylpiperazin in 20 ml absolutem Benzol und erhitzt danach 1 Stunde unter Rückfluß. Das Lösungsmittel wird im Rotationsverdampfer entfernt und der Rückstand mit 70 ml gesättigter Natriumhydrogencarbonat-Lösung versetzt. Man extrahiert 3 mal mit 100 ml Chloroform, wäscht die organische Phase mit Wasser, trocknet und engt im Vakuum ein. Der verbleibende Rückstand wird an neutralem Kieselgel mit Chloroform/Methanol (8:2) als Elutionsmittel chromatographiert. Ausbeute: 1,2 g vom Schmp. 203-205°C (Z.).

Durch Umsetzung mit den entsprechenden Säuren werden die folgenden Salze erhalten: das Dihydrochlorid vom Schmp. 198-200°C (Z.), das Fumarat vom Schmp. 195°C (Z.) (aus Isopropanol) und das Maleat vom Schmp. 208°C (aus Isopropanol).

Beispiel 2

11-(Piperidinoacetyl)-5,6-dihydropyrimido[4.5-b][1.5]benzodiazepinon-(5)

Analog Beispiel 1 erhält man aus 5,76 g (0,02 mol) 11-Chloracetyl-5,6-dihydropyrimido[4.5-b][1.5]benzodiazepinon-(5) und 3,4 g (0,04 mol) Piperidin 2 g der Titelverbindung vom Schmp. 250-252°C (Z.).

Beispiel 3

11-Dimethylaminoacetyl-5,6-dihydropyrimido[4.5-b][1.5]benzodiazepinon-(5)

Zu einer gesättigten Lösung von Dimethylamin in absolutem Ethanol gibt man bei 10°C 5.0 g (0.0174 mol) 11-Chloracetyl-5,6-dihydropyrimido[4.5-b]-[1.5]benzodiazepinon-(5), rührt 15 Minuten bei derselben Temperatur weiter und zieht dann die flüchtigen Bestandteile am Rotationsverdampfer ab. Man versetzt den Rückstand mit 50 ml gesättigter Natriumbicarbonat-Lösung, extrahiert mehrfach mit Chloroform, trocknet die organische Phase und engt ein. Der Rückstand wird aus Ethylacetat/Dichlormethan (6:4) umkristallisiert. Ausbeute: 2,2 g vom Schmp. 208-210°C.

Beispiel 4

11-(1-Piperazinylacetyl)-5,6-dihydropyrimido[4.5-b][1.5]benzodiazepinon-(5)

Zu 1.0 g 11-Chloracetyl-5,6-dihydropyrimido[4.5-b][1.5]benzodiazepinon-(5), gelöst in 10 ml wasserfreiem Dioxan, gibt man bei Zimmertemperatur und unter Rühren 1,2 g Piperazin und erhitzt das erhaltene Gemisch für eine Stunde auf 80°C. Zu dem nach dem Entfernen des Lösungsmittels im Vakuum verbleibenden Rückstand gibt man 50 ml einer gesättigten Natriumhydrogencarbonatlösung. Die Lösung wird mehrfach mit Chloroform extrahiert, die organische Phase wird getrocknet und eingeengt. Der Rückstand wird aus Isopropanol umkristallisiert. Ausbeute: 0,4 g vom Schmp. 200°C (Z.).

Beispiel 5

11-[N-(2-Dimethylaminoethyl)-N-methylamino]-acetyl-5,6-dihydropyrimido-[4.5-b][1.5]benzodiazepinon-(5)

Analog Beispiel 4 erhält man aus 3,0 g 11-Chloracetyl-5,6-dihydropyrimido-[4.5-b][1.5]benzodiazepinon-(5) und 2,6 g N,N,N'-Trimethylethylendiamin in 20 ml absolutem Dioxan 0,45 g der Titelverbindung. Das Dihydrochlorid der Titelverbindung schmilzt bei 184-186°C.

Beispiel 6

11-[(N-Ethyl-n-butylamino)-acetyl]-5,6-dihydropyrimido[4.5-b][1.5]-benzodiazepinon-(5)

Analog Beispiel 4 erhält man aus 3,7 g 11-Chloracetyl-5,6-dihydropyrimido[4.5-b][1.5]benzodiazepinon-(5) und 2,6 g N-Ethyl-n-butylamin in 20 ml absolutem Dioxan durch 2-stündiges Rühren der Mischung bei 70°C, Einengen und Chromatographieren an neutralem Kieselgel mit Methylenchlorid/Methanol (95:5) als Laufmittel 2,2 g der Titelverbindung vom Schmp. 151-152°C (aus Isopropanol).

Beispiel 7

11-Morpholinoacetyl-5,6-dihydropyrimido[4.5-b][1.5]benzodiazepinon-(5)

Analog Beispiel 4 erhält man aus 3,6 g 11-Chloracetyl-5,6-dihydropyrimido[4.5-b][1.5]benzodiazepinon-(5) und 2,2 g Morpholin in 20 ml absolutem Dioxan nach 6-stündigem Rühren der Mischung bei 50°C 2,2 g der Titelverbindung vom Schmp.240-242°C (aus Ethanol). Das Dihydrochlorid der Titelverbindung schmilzt bei 200°C (Z.) (aus Isopropanol).

Beispiel 8

6-Methyl-11-[(4-methyl-1-piperazinyl)-acetyl]-5,6-dihydropyrimido[4.5-b]-[1.5]benzodiazepinon-(5)

Zur Suspension von 4,0 g (0,0133 mol) 11-Chloracetyl-6-methyl-5,6-dihydropyrimido[4.5-b][1.5]benzodiazepinon-(5) in 30 ml absolutem Benzol tropft man bei 50°C innerhalb von 15 Minuten 2,6 g (0,0266 mol) N-Methylpiperazin und rührt noch 1,5 Stunden bei 70°C nach. Man läßt abkühlen, gießt in 100 ml gesättigte Natriumbicarbonatlösung und extrahiert einmal mit 50 ml Ethylacetat und zweimal mit je 100 ml Chloroform. Nach dem Trocknen wird die organische Phase im Vakuum eingeengt und der flüssige Rückstand mit Ether zur Kristallisation gebracht. Die Reinigung erfolgt mittels Filtration durch neutrales Kieselgel (Elution mit Chloroform) und Eindampfen des Filtrats. Ausbeute: 2,7 g vom Schmp. 183-185°C.

Beispiel 9

6-Methyl-11-piperidinoacetyl-5,6-dihydropyrimido[4.5-b][1.5]benzodiazepinon-(5)

Analog Beispiel 8 erhält man aus 2,3 g (0.0076 mol) 11-Chloracetyl-6-methyl-5,6-dihydropyrimido[4.5-b][1.5]benzodiazepinon-(5) in 15 ml absolutem Benzol und 1,94 g (0,0228 mol) Piperidin in 5 ml absolutem Benzol 1,8 g der Titelverbindung vom Schmp. 203-204°C.

Beispiel 10

11-[(N-Ethyl-n-butylamino)-acetyl]-6-methyl-5,6-dihydropyrimido[4.5-b]-[1.5]benzodiazepinon-(5)

Analog Beispiel 8 erhält man aus 1,7 g 11-Chloracetyl-6-methyl-5,6-dihydropyrimido[4.5-b][1.5]benzodiazepinon-(5) und 1,14 g N-Ethyl-n-butylamin nach 2,5-stündigem Rückflußkochen in 20 ml absolutem Benzol 1,5 g der Titelverbindung vom Schmp. 131-133°C (aus Isopropanol).

Beispiel 11

6-Methyl-11-(1-perhydroazepinylacetyl)-5,6-dihydropyrimido[4.5-b][1.5]-benzodiazepinon-(5)

Analog Beispiel 8 erhält man aus 0,5 g 11-Chloracetyl-6-methyl-5,6-dihydropyrimido[4.5-b][1.5]benzodiazepinon-(5) und 0,34 g Perhydroazepin nach 1-stündigem Rückflußkochen in 20 ml absolutem Benzol 0,6 g der Titelverbindung vom Schmp. 206-207°C (aus Isopropanol).

Beispiel 12

6-n-Butyl-11-[(4-methyl-1-piperazinyl)-acetyl]-5,6-dihydropyrimido[4.5-b]-[1.5]benzodiazepinon-(5)

Analog Beispiel 8 erhält man aus 3,5 g 6-n-Butyl-11-chloracetyl-5,6-dihydropyrimido[4.5-b][1.5]benzodiazepinon-(5) und 3.0 g N-Methylpiperazin nach 2,5-stündigem Erhitzen auf 80°C in 140 ml absolutem Toluol 2,0 g der Titelverbindung vom Schmp. 153-154°C (aus Isopropanol).

Beispiel 13

6-n-Butyl-11-morpholinoacetyl-5,6-dihydropyrimido[4.5-b][1.5]benzodiazepinon-(5)

Analog Beispiel 8 erhält man aus 3,5 g 6-n-Butyl-11-chloracetyl-5,6-dihydropyrimido[4.5-b][1.5]benzodiazepinon-(5) und 2,65 g Morpholin nach 3,5-stündigem Erhitzen auf 80°C in 40 ml absolutem Toluol 2,0 g der Titelverbindung vom Schmp. 127-128°C (aus Isopropanol). Das Hydrochlorid der Titelverbindung schmilzt bei 195°C (Z.) (aus Isopropanol).

Beispiel 14

6-Isopropyl-11-[(4-methyl-1-piperazinyl)-acetyl]-5,6-dihydropyrimido-[4.5-b][1.5]benzodiazepinon-(5)

Analog Beispiel 8 erhält man aus 2,8 g 11-Chloracetyl-6-isopropyl-5,6-dihydropyrimido[4.5-b][1.5]benzodiazepinon-(5) und 1,7 g N-Methylpiperazin nach 1-stündigem Erhitzen auf 80°C in 20 ml absolutem Dioxan 1,7 g der Titelverbindung vom Schmp. 133-135°C (aus Ethylacetat/Petrolether).

Beispiel 15

11-Diallylaminoacetyl-6-isopropyl-5,6-dihydropyrimido-[4.5-b][1.5]benzodiazepinon-(5)

Analog Beispiel 8 erhält man aus 2,8 g 11-Chloracetyl-6-isopropyl-5,6-dihydropyrimido[4.5-b][1.5]benzodiazepinon-(5) und 1,7 g Diallylamin nach 5-stündigem Erhitzen auf 100°C in 20 ml absolutem Dioxan 2,0 g der Titelverbindung (Öl). Das Hydrochlorid der Titelverbindung schmilzt bei 205°C (Z.).

Beispiel 16

6-Methyl-11-[(4-methyl-1-piperazinyl)-acetyl]-2-phenyl-5,6-dihydropyrimido[4.5-b][1.5]benzodiazepinon-(5)

Eine Mischung aus 3,3 g (8,7 mmol) 11-Chloracetyl-6-methyl-2-phenyl-5,6-dihydropyrimido[4.5-b][1.5]benzodiazepinon-(5), 1,9 ml (17,4 mmol) N-Methylpiperazin und 40 ml absolutem Toluol wird 2 Stunden auf 70-80$^oC$ erhitzt. Nach dem Abkühlen wird die Reaktionsmischung mit 40 ml Wasser verrührt. Die organische Phase wird mit Natriumsulfat getrocknet und eingedampft. Das zurückbleibende gelbe Kristallisat wird mit Diethylether ausgerührt. Ausbeute: 2,2 g vom Schmp. 234-235$^oC$ (nach Heißextraktion mit Ethanol).

Auflösung des Produktes in halbkonzentrierter Salzsäure und Einengen zur Trockne liefert das Dihydrochlorid der Titelverbindung vom Schmp. 198$^oC$ (Z.) (aus Methanol).

Beispiel 17

6-Methyl-2-phenyl-11-pyrrolidinoacetyl-5,6-dihydropyrimido[4.5-b][1.5]-benzodiazepinon-(5)

1,35 g (19 mmol) Pyrrolidin werden langsam und unter Rühren bei 40$^oC$ zu einer Lösung von 3 g (7 mmol) 11-Chloracetyl-6-methyl-2-phenyl-5,6-dihydropyrimido[4.5-b][1.5]benzodiazepinon-(5) in 30 ml absolutem Toluol gegeben. Die Mischung wird weitere 6 Stunden bei 60$^oC$ gerührt. Man filtriert von festen Bestandteilen ab, engt im Vakuum ein und reinigt den Rückstand durch Chromatographie an neutralem Kieselgel mit Chloroform/Methanol (99:1) als Laufmittel. Nach erneutem Einengen wird der Rückstand mit Methanol aufgerührt und schließlich aus Ethanol umkristallisiert. Ausbeute: 1,1 g (34 % d. Th.) vom Schmp. 196$^oC$.

Beispiel 18

6-Methyl-2-phenyl-11-piperidinoacetyl-5,6-dihydropyrimido[4.5-b][1.5]-benzodiazepinon-(5)

Analog Beispiel 17 erhält man aus 9,1 g (24 mmol) 11-Chloracetyl-6-methyl-2-phenyl-5,6-dihydropyrimido[4.5-b][1.5]benzodiazepinon-(5) und 5,1 g (60 mmol) Piperidin nach 6,5-stündigem Erhitzen auf 110$^oC$ in 180 ml absolutem Toluol 4,6 g (45 %) der Titelverbindung vom Schmp. 203-205$^oC$ (aus Methanol).

Auflösung des Produktes in halbkonzentrierter Salzsäure und Einengen zur Trockne liefert das Dihydrochlorid der Titelverbindung vom Schmp. 165-168$^oC$ (Z.) (aus Ethanol).

Beispiel 19

11-[(N-Ethyl-n-butylamino)-acetyl]-6-methyl-2-phenyl-5,6-dihydropyrimido-[4.5-b][1.5]benzodiazepinon-(5)

Analog Beispiel 17 erhält man aus 11,4 g (30 mmol) 11-Chloracetyl-6-methyl-2-phenyl-5,6-dihydropyrimido[4.5-b][1.5]benzodiazepinon-(5) und 7,6 g (75 mmol) N-Ethyl-n-butylamin nach 7-stündigem Erhitzen auf 110°C in 200 ml absolutem Toluol 4,5 g (34 % d.Th.) der Titelverbindung vom Schmp. 107 - 109°C (aus Methanol).

Beispiel 20

11-Diallylaminoacetyl-6-methyl-2-phenyl-5,6-dihydropyrimido[4.5-b][1.5]-benzodiazepinon-(5)

Analog Beispiel 17 erhält man aus 2,4 g (6 mmol) 11-Chloracetyl-6-methyl-2-phenyl-5,6-dihydropyrimido[4.5-b][1.5]benzodiazepinon-(5) und 1,46 g (15 mmol) Diallylamin nach 5-stündigem Erhitzen auf 110°C in 20 ml absolutem Toluol 2,1 g (80 % d.Th.) der Titelverbindung vom Schmp. 139-140°C (aus Isopropanol).

Beispiel 21

6-Methyl-11-(1-perhydroazepinylacetyl)-2-phenyl-5,6-dihydropyrimido-[4.5-b][1.5]benzodiazepinon-(5)

Analog Beispiel 17 erhält man aus 11 g (29 mmol) 11-Chloracetyl-6-methyl-2-phenyl-5,6-dihydropyrimido[4.5-b][1.5]benzodiazepinon-(5) und 7,3 g (73 mmol) Perhydroazepin nach 7-stündigem Erhitzen auf 110°C in 200 ml absolutem Toluol 9,4 g (73 % d.Th.) der Titelverbindung vom Schmp. 215-216°C (aus Ethanol).

Beispiel 22

6-Methyl-11-morpholinoacetyl-2-phenyl-5,6-dihydropyrimido[4.5-b][1.5]-benzodiazepinon-(5)

Analog Beispiel 17 erhält man aus 11 g (29 mmol) 11-Chloracetyl-6-methyl-2-phenyl-5,6-dihydropyrimido[4.5-b][1.5]benzodiazepinon-(5) und 6,4 g (73 mmol) Morpholin nach 7-stündigem Erhitzen auf 100°C in 200 ml absolutem Toluol 9,6 g (77 % d.Th.) der Titelverbindung vom Schmp. 180-181°C (aus Ethanol).

Beispiel 23

11-[(4-Benzyl-1-piperazinyl)-acetyl]-6-methyl-2-phenyl-5,6-dihydropyrimido[4.5-b][1.5]benzodiazepinon-(5)

Analog Beispiel 17 erhält man aus 10 g (26 mmol) 11-Chloracetyl-6-methyl-2-phenyl-5,6-dihydropyrimido[4.5-b][1.5]benzodiazepinon-(5) und 13,9 g (79 mmol) N-Benzylpiperazin nach 7-stündigem Erhitzen auf 110°C in 200 ml absolutem Toluol 9,5 g (70 % d.Th.) der Titelverbindung vom Schmp. 194°C.

Beispiel 24

11-[(N-n-Butyl-N-tert.-butylamino)-acetyl]-6-methyl-2-phenyl-5,6-dihydropyrimido[4.5-b][1.5]benzodiazepinon-(5)

Analog Beispiel 17 erhält man aus 11,4 g (30 mmol) 11-Chloracetyl-6-methyl-2-phenyl-5,6-dihydropyrimido[4.5-b][1.5]benzodiazepinon-(5) und 11.6 g N-n-Butyl-tert.-butylamin nach 140-stündigem Erhitzen auf 110°C in 200 ml absolutem Toluol 1,0 g der Titelverbindung vom Schmp. 111-113°C.

Beispiel 25

11-[N-(2-Dimethylaminoethyl)-N-methylamino]-acetyl-6-methyl-2-phenyl-5,6-dihydropyrimido[4.5-b][1.5]-benzodiazepinon-(5)

Analog Beispiel 17 erhält man aus 2,1 g (5,5 mmol) 11-Chloracetyl-6-methyl-2-phenyl-5,6-dihydropyrimido[4.5-b][1.5]benzodiazepinon-(5) und 1,43 g (14 mmol) N,N,N'-Trimethyl-ethylendiamin nach 4-stündigem Erhitzen auf 110°C in 40 ml absolutem Toluol 0,6 g (25 % d.Th.) der Titelverbindung vom Schmp. 125°C (aus Isopropanol).

Beispiel 26

11-Dimethylaminoacetyl-6-methyl-2-phenyl-5,6-dihydropyrimido[4.5-b][1.5]-benzodiazepinon-(5)

Analog Beispiel 17 erhält man aus 5,5 g (15 mmol) 11-Chloracetyl-6-methyl-2-phenyl-5,6-dihydropyrimido[4.5-b][1.5]benzodiazepinon-(5) und 11 g (98 mmol) Dimethylamin (in Form einer 40 %igen wäßrigen Lösung) nach 7-stündigem Erhitzen auf 40°C in 55 ml Methylenchlorid 2,8 g (48 % d.Th.) der Titelverbindung vom Schmp. 207-208°C (aus Methanol).

Beispiel 27

11-(Diisopropylaminoacetyl-6-ethyl-2-phenyl-5,6-dihydropyrimido[4.5-b]-[1.5]benzodiazepinon-(5)

Analog Beispiel 17 erhält man aus 9 g (23 mmol) 11-Chloracetyl-6-ethyl-2-phenyl-dihydropyrimido[4.5-b][1.5]benzodiazepinon-(5) und 10 g (100 mmol) Diisopropylamin nach 80-stündigem Rückflußkochen in 150 ml absolutem Dioxan unter Zusatz einer Spatelspitze Kaliumiodid 5,2 g (50 % d.Th.) der Titelverbindung vom Schmp. 120-121°C (aus Cyclohexan).

Beispiel 28

11-[(N-n-Butyl-N-tert.-butylamino)-acetyl]-6-ethyl-2-phenyl-5,6-dihydropyrimido[4.5-b][1.5]benzodiazepinon-(5)

Analog Beispiel 17 erhält man aus 9 g (23 mmol) 11-Chloracetyl-6-ethyl-2-phenyl-5,6-dihydropyrimido[4.5-b][1.5]benzodiazepinon-(5) und 16,6 g (129 mmol) N-n-Butyl-tert.-butylamin nach 58-stündigem Rückflußkochen in 150 ml absolutem Dioxan unter Zusatz einer Spatelspitze Kaliumiodid 3,5 g (33 % d.Th.) der Titelverbindung vom Schmp. 70-72°C.

Beispiel 29

11-[(4-Methyl-1-piperazinyl)-acetyl]-2-phenyl-6-n-propyl-5,6-dihydropyrimido[4.5-b][1.5]benzodiazepinon-(5)

Analog Beispiel 17 erhält man aus 2,5 g (6 mmol) 11-Chloracetyl-2-phenyl-6-n-propyl-5,6-dihydropyrimido[4.5-b][1.5]benzodiazepinon-(5) und 1 g (10 mmol) N-Methylpiperazin nach 5-stündigem Erhitzen auf 50°C in 30 ml absolutem Dioxan 2,3 g (80 % d.Th.) der Titelverbindung vom Schmp. 148-150°C (aus Isopropanol).

Beispiel 30

6-Isopropyl-11-[(4-methyl-1-piperazinyl)-acetyl]-2-phenyl-5,6-dihydropyrimido[4.5-b][1.5]benzodiazepinon-(5)

Analog Beispiel 17 erhält man aus 12,5 g (31 mmol) 11-Chloracetyl-6-isopropyl-2-phenyl-5,6-dihydropyrimido[4.5-b][1.5]benzodiazepinon-(5) und 10 g (100 mmol) N-Methylpiperazin nach 6-stündigem Rückflußkochen in 120 ml absolutem Dioxan 11,5 g (80 % d.Th.) der Titelverbindung vom Schmp. 168-170°C (aus Aceton).

Beispiel 31

6-Isopropyl-2-phenyl-11-piperidinoacetyl-5,6-dihydropyrimido[4.5-b][1.5]-benzodiazepinon-(5)

Analog Beispiel 17 erhält man aus 7 g (17 mmol) 11-Chloracetyl-6-isopropyl-2-phenyl-5,6-dihydropyrimido[4.5-b][1.5]benzodiazepinon-(5) und 3 g (35 mmol) Piperidin nach 16-stündigem Rückflußkochen in 70 ml absolutem Dioxan 5,85 g (75 % d.Th.) der Titelverbindung vom Schmp. 152-154°C (aus Isopropanol).

Beispiel 32

6-Isopropyl-11-(1-perhydroazepinylacetyl)-2-phenyl-5,6-dihydropyrimido-[4.5-b][1.5]benzodiazepinon-(5)

Analog Beispiel 17 erhält man aus 5 g (12,5 mmol) 11-Chloracetyl-6-isopropyl-2-phenyl-5,6-dihydropyrimido[4.5-b][1.5]benzodiazepinon-(5) und 2,5 g (25 mmol) Perhydroazepin nach 3-stündigem Rückflußkochen in 50 ml absolutem Toluol 3,6 g (63 % d.Th.) der Titelverbindung vom Schmp. 180-182°C

Beispiel 33

6-n-Butyl-11-[(4-methyl-1-piperazinyl)-acetyl]-2-phenyl-5,6-dihydropyrimido[4.5-b][1.5]benzodiazepinon-(5)

Analog Beispiel 17 erhält man aus 10 g (24 mmol) 6-n-Butyl-11-chloracetyl-2-phenyl-5,6-dihydropyrimido[4.5-b][1.5]benzodiazepinon-(5) und 10 g (100 mmol) N-Methylpiperazin nach 4-stündigem Rückflußkochen in 100 ml absolutem Dioxan 4,4 g (38 % d.Th.) der Titelverbindung vom Schmp. 90-94°C (aus Aceton).

Beispiel 34

6-n-Butyl-11-diallylaminoacetyl-2-phenyl-5,6-dihydropyrimido[4.5-b][1.5]-benzodiazepinon-(5)

Analog Beispiel 17 erhält man aus 8 g (19 mmol) 6-n-Butyl-11-chloracetyl-2-phenyl-5,6-dihydropyrimido[4.5-b][1.5]benzodiazepinon-(5) und 9,7 g (100 mmol) Diallylamin nach 5-stündigem Rückflußkochen in 150 ml absolutem Dioxan 6,7 g (73 % d.Th.) der Titelverbindung vom Schmp. 137-138°C (aus Cyclohexan).

Beispiel 35

6-n-Butyl-11-di-n-butylaminoacetyl-2-phenyl-5,6-dihydropyrimido[4.5-b]-[1.5]benzodiazepinon-(5)

Analog Beispiel 17 erhält man aus 8 g (19 mmol) 6-n-Butyl-11-chloracetyl-2-phenyl-5,6-dihydropyrimido[4.5-b][1.5]benzodiazepinon-(5) und 12,9 g (100 mmol) Di-n-butylamin nach 4-stündigem Rückflußkochen in 150 ml absolutem Dioxan 6,6 g (68 % d.Th.) der Titelverbindung vom Schmp. 103-104°C (aus Cyclohexan). Das Fumarat schmilzt bei 114-115°C.

Beispiel 36

6-sec.-Butyl-11-[(4-methyl-1-piperazinyl)-acetyl]-2-phenyl-5,6-dihydropyrimido[4.5-b][1.5]benzodiazepinon-(5)

Analog Beispiel 17 erhält man aus 4,3 g (10 mmol) 6-sec.-Butyl-11-chloracetyl-2-phenyl-5,6-dihydropyrimido[4.5-b][1.5]benzodiazepinon-(5) und 5 g (50 mmol) N-Methyl-piperazin nach 5-stündigem Rückflußkochen in 40 ml absolutem Dioxan 2,6 g (53 % d.Th.) der Titelverbindung vom Schmp. 153-154°C (aus Isopropanol).

Beispiel 37

11-(1-Homopiperazinylacetyl)-6-isobutyl-2-phenyl-5,6-dihydropyrimido-[4.5-b][1.5]benzodiazepinon-(5)

Analog Beispiel 17 erhält man aus 5,5 g (13 mmol) 11-Chloracetyl-6-isobutyl-2-phenyl-5,6-dihydropyrimido[4.5-b][1.5]benzodiazepinon-(5), 1,3 g (13 mmol) Homopiperazin und 1,3 g Triethylamin nach 6-stündigem Erhitzen auf 50°C in 50 ml absolutem Dioxan 0,4 g der Titelverbindung vom Schmp. 118-120°C (aus Isopropanol).

Beispiel 38

6-Isobutyl-11-piperidinoacetyl-2-phenyl-5,6-dihydropyrimido[4.5-b][1.5]-benzodiazepinon-(5)

Analog Beispiel 17 erhält man aus 7 g (16,6 mmol) 11-Chloracetyl-6-isobutyl-2-phenyl-5,6-dihydropyrimido[4.5-b][1.5]benzodiazepinon-(5) und 8,5 g (100 mmol) Piperidin nach 3-stündigem Rückflußkochen in 70 ml absolutem Dioxan 3,5 g (45 % d.Th.) der Titelverbindung vom Schmp. 193-194°C (aus Isopropanol).

Beispiel 39

6-tert.-Butyl-11-piperidinoacetyl-2-phenyl-5,6-dihydropyrimido[4.5-b]-[1.5]benzodiazepinon-(5)

Analog Beispiel 17 erhält man aus 6,3 g (15 mmol) 6-tert.-Butyl-11-chloracetyl-2-phenyl-5,6-dihydropyrimido[4.5-b][1.5]benzodiazepinon-(5) und 8.5 g (100 mmol) Piperidin nach 3-stündigem Rückflußkochen in 70 ml absolutem Dioxan 1.8 g (26 % d.Th.) der Titelverbindung vom Schmp. 193-194°C (aus Isopropanol).

Beispiel 40

6-Methyl-2-phenyl-11-[2-(piperidino)-propionyl]-5,6-dihydropyrimido-[4.5-b][1.5]benzodiazepinon-(5)

Analog Beispiel 17 erhält man aus 4,5 g (10 mmol) 11-[2-(Brompropionyl)]-6-methyl-2-phenyl-5,6-dihydropyrimido[4.5-b][1.5]benzodiazepinon-(5) und 1,7 g (20 mmol) Piperidin nach 4-stündigem Erhitzen auf 110°C in 90 ml absolutem Toluol 2 g (45 % d.Th.) der Titelverbindung vom Schmp. 214-215°C (aus Ethanol).

Beispiel 41

6-Methyl-2-phenyl-11-[2-(piperazino)-propionyl]-5,6-dihydropyrimido-[4.5-b][1.5]benzodiazepinon-(5)

Analog Beispiel 17 erhält man aus 4,5 g ( 10 mmol) 11-[2-(Brompropionyl)]-6-methyl-2-phenyl-5,6-dihydropyrimido[4.5-b][1.5]benzodiazepinon-(5) und 1,76 g (20 mmol) Piperazin nach 10-stündigem Erhitzen auf 110°C in 90 ml absolutem Toluol 0,3 g der Titelverbindung vom Schmp. 197°C (aus Isopropanol).

Beispiel 42

6-Methyl-11-[(4-methyl-1-piperazinyl)-acetyl]-2-phenyl-5,6-dihydropyrimido[4.5-b][1.5]benzodiazepinon-(5)-N-oxid

Eine Lösung von 0,7 g (3,4 mmol) m-Chlorperbenzoesäure (85 %ig) in 10 ml Methanol wird langsam bei Raumtemperatur zu einer Suspension von 1g (2,3 mmol) 6-Methyl-11-[(4-methyl-1-piperazinyl)-acetyl]-2-phenyl-5,6-diyhdropyrimido[4.5-b][1.5]benzodiazepinon-(5) in 40 ml Methanol gegeben. Nach 3-stündigem Rühren bei Raumtemperatur wird abfiltriert und das Filtrat im Vakuum eingeengt. Der Rückstand wird an neutralem Kieselgel mit Chloroform/Methanol (20:1) als Laufmittel chromatographiert. Ausbeute: 0,5 g (48 % d.Th.).

Beispiel 43

11-Chloracetyl-5,6-dihydropyrimido[4.5-b][1.5]benzodiazepinon-(5)

In die Suspension von 4,24 g (0,02 mol) 5,6-Dihydropyrimido[4.5-b][1.5]-benzodiazepinon-(5) in 60 ml absolutem Dioxan tropft man unter Rühren bei 60-70°C innerhalb von 20 Minuten gleichzeitig und möglichst gleichmäßig eine Lösung von 3,16 g (0,028 mol) Chloracetylchlorid in 8 ml absolutem Dioxan sowie eine Lösung von 2,82 g (0,028 mol) trockenem Triethylamin in 6 ml absolutem Dioxan. Man erhitzt noch 4 Stunden unter Rückfluß,

saugt vom ausgefallenen Triethylaminhydrochlorid ab und engt das Filtrat im Vakuum ein. Der Rückstand wird mit Chloroform an neutralem Kieselgel chromatographiert, das Eluat eingedampft und dessen Rückstand mit Ether verrührt und nach Filtrieren aus Ethanol umkristallisiert. Ausbeute: 3,9 g vom Schmp. 196°C (Z.) (aus Isopropanol).

Beispiel 44

11-Chloracetyl-6-methyl-5,6-dihydropyrimido[4.5-b][1.5]benzodiazepinon-(5)

Zur Suspension von 4,6 g (0,020 mol) 6-Methyl-5,6-dihydropyrimido-[4.5-b][1.5]benzodiazepinon-(5) in 35 ml absolutem Dioxan werden innerhalb von 20 Minuten bei 80°C gleichzeitig 6,25 g (0,044 mol) Chloracetylchlorid in 15 ml absolutem Dioxan und 4,4 ml trockenes Pyridin in 15 ml Dioxan getropft. Man erhitzt noch 15 Minuten zum Sieden und läßt dann abkühlen. Von der schweren dunklen Phase wird abdekantiert, diese mit Dichlormethan gewaschen und die Dioxan- und Dichlormethanphasen im Vakuum eingeengt. Das verbleibende Öl wird, gelöst in wenig Chloroform, über neutrales Kieselgel filtriert, das Filtrat eingedampft und der Rückstand aus Isopropanol umkristallisiert. Ausbeute: 2,8 g vom Schmp. 176-178°C (Z.).

Beispiel 45

6-n-Butyl-11-chloracetyl-5,6-dihydropyrimido[4.5-b][1.5]benzodiazepinon-(5)

Analog Beispiel 44 erhält man aus 6-n-Butyl-5,6-dihydropyrimido[4.5-b]-[1.5]benzodiazepinon-(5) und Chloracetylchlorid nach 2-stündigem Rückflußkochen in absolutem Dioxan unter Verwendung von Triethylamin als Base die ölige Titelverbindung.

Beispiel 46

11-Chloracetyl-6-isopropyl-5,6-dihydropyrimido[4.5-b][1.5]benzodiazepinon-(5)

Analog Beispiel 44 erhält man aus 6-Isopropyl-5,6-dihydropyrimido[4.5-b]-[1.5]benzodiazepinon-(5) und Chloracetylchlorid nach 2-stündigem Rückflußkochen in absolutem Dioxan unter Verwendung von Triethylamin als Base die ölige Titelverbindung.

Beispiel 47

11-Chloracetyl-6-methyl-2-phenyl-5,6-dihydropyrimido[4.5-b][1.5]benzodiazepinon-(5)

Zur Suspension von 3 g (10 mmol) 6-Methyl-2-phenyl-5,6-dihydropyrimido-[4.5-b][1.5]benzodiazepinon-(5) in 30 ml absolutem Toluol tropft man bei Raumtemperatur 1,6 ml (20 mmol) Chloracetylchlorid und erhitzt anschliessend 3 Stunden unter Rückfluß. Nach dem Abkühlen wird vom ausgefallenen Niederschlag abgesaugt und das Filtrat eingedampft. Der Rückstand wird mit Diethylether ausgerührt. Ausbeute: 3,3 g (86 % d.Th.) vom Schmp. 189-190°C (aus Methylethylether).

Beispiel 48

11-Chloracetyl-6-ethyl-2-phenyl-5,6-dihydropyrimido[4.5-b][1.5]benzodiazepinon-(5)

28 g (250 mmol) Chloracetylchlorid und 28 g (250 mmol) Natriumcarbonat werden zu einer Lösung von 20 g (63 mmol) 6-Ethyl-2-phenyl-5,6-dihydropyrimido[4.5-b][1.5]benzodiazepinon-(5) in 500 ml absolutem Toluol gegeben. Die Mischung wird 20 Stunden bei 100°C gerührt. Das Lösungsmittel wird im Vakuum abgezogen, und der Rückstand wird mit 2n Salzsäure neutralisiert. Nach Extraktion mit Chloroform wird die organische Phase mit Wasser gewaschen, getrocknet und im Vakuum eingeengt. Der zurückbleibende Rückstand wird aus Isopropanol umkristallisiert. Ausbeute: 19,2 g (77 % d.Th.) vom Schmp. 147-148°C.

Beispiel 49

11-Chloracetyl-2-phenyl-6-n-propyl-5,6-dihydropyrimido[4.5-b][1.5]benzodiazepinon-(5)

Analog Beispiel 48 erhält man aus 5,8 g (17.6 mmol) 2-Phenyl-6-n-propyl-5,6-dihydropyrimido[4.5-b][1.5]benzodiazepinon-(5) und 5,7 g (50 mmol) Chloracetylchlorid nach 65 Stunden Reaktionszeit 3,2 g (45 % d.Th.) der Titelverbindung vom Schmp. 139-140°C (aus Isopropanol).

Beispiel 50

11-Chloracetyl-6-isopropyl-2-phenyl-5,6-dihydropyrimido[4.5-b][1.5]benzodiazepinon-(5)

Analog Beispiel 48 erhält man aus 15 g (45 mmol) 6-Isopropyl-2-phenyl-5,6-dihydropyrimido[4.5-b][1.5]benzodiazepinon-(5) und 23 g (200 mmol) Chloracetylchlorid nach 60 Stunden Reaktionszeit 13,5 g (76 % d.Th.) der Titelverbindung vom Schmp. 204-205°C (aus Isopropanol).

Beispiel 51

6-n-Butyl-11-chloracetyl-2-phenyl-5,6-dihydropyrimido[4.5-b][1.5]benzodiazepinon-(5)

Analog Beispiel 48 erhält man aus 14 g (41 mmol) 6-n-Butyl-2-phenyl-5,6-dihydropyrimido[4.5-b][1.5]benzodiazepinon-(5) und 15 g (130 mmol) Chloracetylchlorid nach 24 Stunden Reaktionszeit 16,1 g (94 % d.Th.) der Titelverbindung vom Schmp. 139-140°C (aus Isopropanol).

Beispiel 52

11-Chloracetyl-6-isobutyl-2-phenyl-5,6-dihydropyrimido[4.5-b][1.5]benzodiazepinon-(5)

Analog Beispiel 48 erhält man aus 14 g (41 mmol) 6-Isobutyl-2-phenyl-5,6-dihydropyrimido[4.5-b][1.5]benzodiazepinon-(5) und 15 g (130 mmol) Chloracetylchlorid nach 26 Stunden Reaktionszeit 15,3 g (89 %) der Titelverbindung vom Schmp. 95 - 96°C (aus Isopropanol).

Beispiel 53

6-sec.-Butyl-11-chloracetyl-2-phenyl-5,6-dihydropyrimido[4.5-b][1.5]-benzodiazepinon-(5)

Analog Beispiel 48 erhält man aus 3,9 g (11,3 mmol) 6-sec.-Butyl-2-phenyl-5,6-dihydropyrimido[4.5-b][1.5]benzodiazepinon-(5) und 3,4 g (30 mmol) Chloracetylchlorid nach 15 Stunden Reaktionszeit 3,8 g (80 %) der Titelverbindung vom Schmp. 170-171°C (aus Isopropanol).

Beispiel 54

6-tert.-Butyl-11-chloracetyl-2-phenyl-5,6-dihydropyrimido[4.5-b][1.5]benzodiazepinon-(5)

Analog Beispiel 48 erhält man aus 14 g (41 mmol) 6-tert.-Butyl-2-phenyl-5,6-dihydropyrimido[4.5-b][1.5]benzodiazepinon-(5) und 17 g (150 mmol) Chloracetylchlorid nach 12 Stunden Reaktionszeit 13 g (75 % d.Th.) der Titelverbindung vom Schmp. 176-177°C (aus Isopropanol).

Beispiel 55

11-(2-Brompropionyl)-6-methyl-2-phenyl-5,6-dihydropyrimido[4.5-b][1.5]-benzodiazepinon-(5)

6,8 g (23 mmol) 6-Methyl-2-phenyl-5,6-dihydropyrimido[4.5-b][1.5]benzodiazepinon-(5) und 7,9 g (46 mmol) 2-Brompropionylchlorid werden in 70 ml absolutem Toluol 68 Stunden unter Rückfluß erhitzt. Von einem geringen Niederschlag wird abfiltriert und das Lösungsmittel am Rotationsverdampfer entfernt. Nach Animpfen und Ausrühren des verbleibenden zähen Öls mit Aceton erhält man 8,3 g beigefarbenes Produkt, welches aus Ethylmethylketon umkristallisiert wird. Schmp. 215°C.

Beispiel 56

6-Methyl-5,6-dihydropyrimido[4.5-b][1.5]benzodiazepinon-(5)

a) 34,4 g (0,125 mol) 5-Ethoxycarbonyl-4-(2-methylaminoanilino)-pyrimidin werden in eine siedende Lösung von 2,9 g (0,125 mol) Natrium in 150 ml absolutem Ethanol eingetragen und der Ansatz 1 Stunde unter Rückfluß gerührt. Man läßt abkühlen, versetzt mit 9,3 ml Eisessig, saugt den Niederschlag ab, wäscht mit Ethanol und trocknet im Vakuum. Ausbeute: 27,5 g vom Schmp. 236-237°C (aus Ethanol).

b) In die Lösung von 0,617 g (0,0055 mol) Kalium-t-butanolat in 5 ml absolutem Dimethylsulfoxid trägt man bei Raumtemperatur unter Rühren 1 g (0,005 mol) 5,6-Dihydropyrimido[4.5-b][1.5]benzodiazepinon-(5) (J.Chem.Soc. C 1968, 1753) ein. Man erwärmt 15 Minuten auf 50°C, rührt anschließend noch 1 Stunde ohne Heizung nach und tropft 0,915 g (0,0065 mol) Methyliodid zu. Es wird 1 Stunde bei Raumtemperatur und 2 Stunden bei 55°C nachgerührt und die Mischung nach Stehen über Nacht in 50 ml Eiswasser eingetragen. Man extrahiert mit Chloroform, trocknet die organische Phase und engt ein. Nach Chromatographie an neutralem Kieselgel mit Chloroform als Elutionsmittel erhält man 0,4 g der Titelverbindung vom Schmp. 236-237°C (aus Ethanol).

Beispiel 57

6-n-Butyl-5,6-dihydropyrimido[4.5-b][1.5]benzodiazepinon-(5)

Die Titelverbindung (Schmp. 145-147°C, aus Ethanol) wird analog Beispiel 56 a) aus 5-Ethoxycarbonyl-4-(2-n-butylaminoanilino)-pyrimidin hergestellt.

Beispiel 58

6-Isopropyl-5,6-dihydropyrimido[4.5-b][1.5]benzodiazepinon-(5)

12,7 g (0,03 mol) 5-Ethoxycarbonyl-4-(2-isopropylaminoanilino)-pyrimidin werden in 42,2 ml einer 0,1n Natronlauge für eine Stunde unter Rückfluß zum Sieden erhitzt. Das Lösungsmittel wird im Vakuum abgezogen, und der Rückstand wird bei 60°C im Vakuum getrocknet. 28,9 g (0,11 mol) Triphenylphosphin und 16,9 g (0,11 mol) Tetrachlorkohlenstoff werden zu dem in 150 ml absoluten Dioxan gelösten trockenen Produkt gegeben, und die Mischung wird 5 Stunden bei 80°C gerührt. Das Lösungsmittel wird abgezogen, und der ölige Rückstand wird an neutralem Kieselgel chromatographiert (Laufmittel: Methylenchlorid/Methanol 99:1). Ausbeute: 6,3 g vom Schmp. 204-206°C (aus Isopropanol).

Beispiel 59

6-Methyl-2-phenyl-5,6-dihydropyrimido[4.5-b][1.5]benzodiazepinon-(5)

In die frisch hergestellte, heiße Lösung von 6,8 g (0,296 mol) Natrium in 99 ml absolutem Ethanol gibt man unter Rühren 34,8 g (0,1 mol) 5-Ethoxycarbonyl-4-(2-methylaminoanilino)-2-phenylpyrimidin. Man erhitzt die dabei gebildete orangefarbene Suspension noch 30 Minuten unter Rückfluß, läßt abkühlen und rührt nach Versetzen mit 19,7 ml Eisessig und 19,4 ml Wasser noch 30 Minuten. Der gelbe Niederschlag wird abgesaugt, mit Wasser gewaschen und im Vakuum getrocknet. Ausbeute 26 g vom Schmp. 227°C (aus Ethanol).

Beispiel 60

6-Ethyl-2-phenyl-5,6-dihydropyrimido[4.5-b][1.5]benzodiazepinon-(5)

Analog Beispiel 59 erhält man aus 31 g (86 mmol) 5-Ethoxycarbonyl-4-(2-ethylaminoanilino)-2-phenylpyrimidin 21 g (77 % d.Th.) der Titelverbindung vom Schmp. 198-199°C (aus Isopropanol).

Beispiel 61

2-Phenyl-6-n-propyl-5,6-dihydropyrimido[4.5-b][1.5]benzodiazepinon-(5)

a) 7,9 g (30 mmol) fein gepulvertes Triphenylphosphin und 4,6 g (30 mmol) Tetrachlorkohlenstoff werden zu einer Lösung von 6,2 g (18 mmol) 4-(2-n-Propylaminoanilino)-2-phenyl-5-pyrimidincarbonsäure in 40 ml absolutem Tetrahydrofuran gegeben. Die Mischung wird 15 Stunden unter Rückfluß zum Sieden erhitzt. Nach Zugabe von 100 ml Wasser wird das Gemisch mit Chloroform extrahiert. Die organische Phase wird mit Wasser

gewaschen, getrocknet und im Vakuum eingeengt. Ausbeute: 2,5 g (85 % d.Th.) vom Schmp. 152-153°C.

b) 2 g (22 %) der Titelverbindung werden analog Beispiel 59 aus 10,5 g (28 mmol) 5-Ethoxycarbonyl-4-(2-propylaminoanilino)-2-phenyl-pyrimidin erhalten.

Beispiel 62

6-Isopropyl-2-phenyl-5,6-dihydropyrimido[4.5-b][1.5]benzodiazepinon-(5)

a) 13,5 g (75 %) der Titelverbindung (Schmp. 195-196°C) erhält man analog Beispiel 61 a) aus 19 g (55 mmol) 4-(2-Isopropylaminoanilino)-2-phenyl-5-pyrimidincarbonsäure, 29,2 g (111 mmol) Triphenylphosphin und 18,6 g (121 mmol) Tetrachlorkohlenstoff.

b) Man erhält die Titelverbindung analog Beispiel 59 aus 5-Ethoxycarbonyl-4-(2-isopropylaminoanilino)-2-phenylpyrimidin.

Beispiel 63

6-n-Butyl-2-phenyl-5,6-dihydropyrimido[4.5-b][1.5]benzodiazepinon-(5)

7.5 g (72 %) der Titelverbindung (Schmp. 140-141°C, aus Isopropanol) erhält man analog Beispiel 59 aus 22 g (56 mmol) 5-Ethoxycarbonyl-4-(2-n-butylaminoanilino)-2-phenylpyrimidin.

Beispiel 64

6-Isobutyl-2-phenyl-5,6-dihydropyrimido[4.5-b][1.5]benzodiazepinon-(5)

15 g (79 %) der Titelverbindung (Schmp. 203-204°C, aus Isopropanol) erhält man analog Beispiel 61 a) aus 20 g (55 mmol) 4-(2-Isobutylaminoanilino)-2-phenyl-5-pyrimidincarbonsäure, 26,2 g (100 mmol) Triphenylphosphin und 15,4 g (100 mmol) Tetrachlorkohlenstoff.

Beispiel 65

6-sec.-Butyl-2-phenyl-5,6-dihydropyrimido[4.5-b][1.5]benzodiazepinon-(5)

6,2 g (73 %) der Titelverbindung (Schmp. 203-205°C aus Isopropanol) erhält man aus 9 g (25 mmol) 4-(2-sec.-Butylaminoanilino)-2-phenyl-5-pyrimidincarbonsäure, 11,5 g (43.8 mmol) Triphenylphosphin und 6,2 g (40 mmol) Tetrachlorkohlenstoff.

Beispiel 66

6-tert.-Butyl-2-phenyl-5,6-dihydropyrimido[4.5-b][1.5]benzodiazepinon-(5)

Man erhält die Titelverbindung (Schmp. 221-222°C, aus Isopropanol) analog Beispiel 61 a) aus 4-(2-tert.-Butylaminoanilino)-2-phenyl-5-pyrimidincarbonsäure.

Beispiel 67

5-Ethoxycarbonyl-4-(2-methylaminoanilino)-pyrimidin

In die siedende Lösung von 48,8 g (0,4 mol) N-Methyl-o-phenylendiamin in 500 ml absolutem Ethylacetat tropft man unter Rühren zuerst 40,4 g (0,4 mol) Triethylamin und anschließend eine Lösung von 74,4 g (0,4 mol) 4-Chlor-5-ethoxycarbonylpyrimidin in 500 ml absolutem Ethylacetat. Man erhitzt danach noch 4 Stunden unter Rückfluß, saugt das ausgefallene Triethylaminhydrochlorid heiß ab und engt das Filtrat ein. Der Rückstand wird aus Isopropanol umkristallisiert. Ausbeute: 34,4 g vom Schmp. 153-155°C.

5-Ethoxycarbonyl-4-(2-n-butylaminoanilino)-pyrimidin
(Schmp. 103-104°C, aus Isopropanol) und
5-Ethoxycarbonyl-4-(2-isopropylaminoanilino)-pyrimidin (Schmp. 103-104°C, aus Isopropanol)
erhält man analog durch Reaktion von
N-n-Butyl-o-phenylendiamin und
N-Isopropyl-o-phenylendiamin mit
4-Chlor-5-ethoxycarbonylpyrimidin.

Beispiel 68

4-(2-n-Propylaminoanilino)-2-phenyl-5-pyrimidincarbonsäure

14,5 g (39 mmol) 5-Ethoxycarbonyl-4-(2-n-propylaminoanilino)-2-phenylpyrimidin und 8,7 g (156 mmol) Kaliumhydroxid (gelöst in 140 ml Ethanol und 60 ml Wasser) werden 5 Stunden unter Rückfluß zum Sieden erhitzt. Man engt im Vakuum zur Trockne ein, säuert den Rückstand mit verdünnter Salzsäure an und extrahiert mit Chloroform. Die organische Phase wird mit Wasser gewaschen, getrocknet und im Vakuum eingeengt. Ausbeute: 5,8 g (46 %) vom Schmp. 205-206°C (aus Isopropanol).

4-(2-Isopropylaminoanilino)-2-phenyl-5-pyrimidincarbonsäure (Schmp. 223-225°C (Z.), aus Isopropanol),
4-(2-Isobutylaminoanilino)-2-phenyl-5-pyrimidincarbonsäure (Schmp. 192-193°C),

4-(2-sec.-Butylaminoanilino)-2-phenyl-5-pyrimidincarbonsäure (Schmp. 202-203°C, aus Isopropanol) und
4-(2-tert.-Butylaminoanilino)-2-phenyl-5-pyrimidincarbonsäure [Monohydrat, Schmp. 247-248°C (Z.), aus Isopropanol]
werden auf gleiche Weise aus den entsprechenden Ethylestern hergestellt.

Beispiel 69

5-Ethoxycarbonyl-4-(2-methylaminoanilino)-2-phenyl-pyrimidin

Zu einer Lösung von 21,5 g (0,175 mol) N-Methyl-o-phenylendiamin und 27 ml (0,19 mol) Triethylamin in 220 ml absolutem Ethanol gibt man bei 60°C in kleinen Portionen 46,2 g (0,176 mol) 4-Chlor-5-ethoxycarbonyl-2-phenylpyrimidin und erhitzt danach noch 1 Stunde unter Rückfluß. Nach dem Abkühlen wird der ausgefallene gelbe Niederschlag abgesaugt, mit insgesamt 100 ml Ethanol gewaschen und getrocknet. Ausbeute 39,4 g vom Schmp. 165°C (aus Ethylacetat).

Analoge Reaktionen in Toluol (6 Stunden unter Rückfluß), Ethylacetat (6 Stunden unter Rückfluß) und Dimethylformamid (8 Stunden unter Rückfluß) ergeben 70 %, 70 % und 90% der Titelverbindung.
5-Ethoxycarbonyl-4-(2-ethylaminoanilino)-2-phenylpyrimidin (Schmp. 135-136°C),
5-Ethoxycarbonyl-4-(2-n-propylaminoanilino)-2-phenylpyrimidin (Schmp. 104-105°C),
5-Ethoxycarbonyl-4-(2-isopropylaminoanilino)-2-phenylpyrimidin (Schmp. 126-127°C),
5-Ethoxycarbonyl-4-(2-n-butylaminoanilino)-2-phenylpyrimidin (Schmp. 112-113°C),
5-Ethoxycarbonyl-4-(2-isobutylaminoanilino)-2-phenylpyrimidin (Schmp. 113-115°C),
5-Ethoxycarbonyl-4-(2-sec.-butylaminoanilino)-2-phenylpyrimidin (Schmp. 94-95°C) und
5-Ethoxycarbonyl-4-(2-tert.-butylaminoanilino)-2-phenylpyrimidin (Schmp. 127-128°C)
werden auf die gleiche Weise durch Umsetzung von
N-Ethyl-o-phenylendiamin,
N-n-Propyl-o-phenylendiamin,
N-Isopropyl-o-phenylendiamin,
N-n-Butyl-o-phenylendiamin,

N-Isobutyl-o-phenylendiamin,
N-sec.-Butyl-o-phenylendiamin und
N-tert.-Butyl-o-phenylendiamin mit
4-Chlor-5-ethoxycarbonyl-2-phenylpyrimidin erhalten.

Beispiel 70

11-[(4-Methyl-1-piperazinyl)-acetyl]-2-phenyl-5,6-dihydropyrimido[4.5-b]-[1.5]benzodiazepinon-(5)

Analog Beispiel 17 erhält man aus 0,35 g (0,96 mmol) 11-Chloracetyl-2-phenyl-5,6-dihydropyrimido[4.5-b][1.5]benzodiazepinon-(5) und 0,27 g (2,7 mmol) N-Methylpiperazin nach 5-stündigem Rückflußkochen in 3,5 ml absolutem Dioxan 0,2 g (52 % d.Th.) der Titelverbindung.

Beispiel 71

11-Piperidinoacetyl-2-phenyl-5,6-dihydropyrimido[4.5-b][1.5]benzodiazepinon-(5)

Analog Beispiel 17 erhält man aus 1,1 g (3 mmol) 11-Chloracetyl-2-phenyl-5,6-dihydropyrimido[4.5-b][1.5]benzodiazepinon-(5) und 0,64 g (7,5 mmol) Piperidin nach 4-stündigem Rückflußkochen in 10 ml absolutem Dioxan 0,58 g (47 % d.Th.) der Titelverbindung.

Beispiel 72

11-Chloracetyl-2-phenyl-5,6-dihydropyrimido[4.5-b][1.5]benzodiazepinon-(5)

Analog Beispiel 47 erhält man aus 5 g (15 mmol) 2-Phenyl-5,6-dihydropyrimido[4.5-b][1.5]benzodiazepinon-(5) und 6.8 g (60 mmol) Chloracetylchlorid nach 7-stündigem Rückflußkochen in 75 ml Toluol 3,3g (60 % d.Th.) der Titelverbindung vom Schmp. 228-232°C.

Beispiel 73

2-Phenyl-5,6-dihydropyrimido[4.5-b][1.5]benzodiazepinon-(5)

78 g 5-Ethoxycarbonyl-4-(2-aminoanilino)-2-phenylpyrimidin werden unter Rühren zu einer frisch bereiteten siedenden Lösung von 8 g (350 mmol) Natrium in 250 ml absolutem Ethanol gegeben. Die sich dabei bildende Suspension wird etwa 45 Minuten bis zur vollständigen Lösung unter Rückfluß zum Sieden erhitzt. Nach dem Abkühlen wird die Mischung mit 20 ml (350 mmol) Eisessig neutralisiert. 200 ml Ethanol und 200 ml Wasser werden zugesetzt und der sich bildende Niederschlag wird abfiltriert, mit Wasser gewaschen und im Vakuum getrocknet. Ausbeute 66 g (97 % d.Th.) vom Schmp.

260-261°C (aus Dimethylformamid/Wasser).

Beispiel 74

5-Ethoxycarbonyl-4-(2-aminoanilino)-2-phenylpyrimidin

Man erhält 49 g (90 %) der Titelverbindung (Schmp. 154-156°C, aus Ethylacetat) analog Beispiel 69 nach 1,5-stündigem Rückflußkochen von 24 g (220 mmol) o-Phenylendiamin, 42,7 g (163 mmol) 4-Chlor-5-ethoxycarbonyl-2-phenylpyrimidin und 23 g (230 mmol) Triethylamin in 420 ml absolutem Ethanol.

Beispiel 75

11-[N-(2-Dimethylaminoethyl)-N-methylamino]-acetyl-2-phenyl-5,6-dihydropyrimido[4.5-b][1.5]benzodiazepinon-(5)

Analog Beispiel 17 erhält man die Titelverbindung aus 11-Chloracetyl-2-phenyl-5,6-dihydropyrimido[4.5-b][1.5]benzodiazepinon-(5) und N,N,N'-Trimethylethylendiamin.

Beispiel 76

11-[N-(2-Dimethylaminoethyl)-N-ethylamino]-acetyl-2-phenyl-5,6-dihydropyrimido[4.5-b][1.5]benzodiazepinon-(5)

Analog Beispiel 17 erhält man die Titelverbindung aus 11-Chloracetyl-2-phenyl-5,6-dihydropyrimido[4.5-b][1.5]benzodiazepinon-(5) und N-Ethyl-N',N'-dimethylethylendiamin.

Beispiel 77

11-[N-(2-Diethylaminoethyl)-N-ethylamino]-acetyl-2-phenyl-5,6-dihydropyrimido[4.5-b][1.5]benzodiazepinon-(5)

Analog Beispiel 17 erhält man die Titelverbindung aus 11-Chloracetyl-2-phenyl-5,6-dihydropyrimido[4.5-b][1.5]benzodiazepinon-(5) und N,N,N'-Triethylethylendiamin.

Beispiel 78

11-[N-(2-Dimethylaminoethyl)-N-ethylamino]-acetyl-6-methyl-2-phenyl-5,6-dihydropyrimido[4.5-b][1.5]benzodiazepinon-(5)

Analog Beispiel 17 erhält man die Titelverbindung aus 11-Chloracetyl-6-methyl-2-phenyl-5,6-dihydropyrimido[4.5-b][1.5]benzodiazepinon-(5) und N-Ethyl-N',N'-dimethylethylendiamin.

Beispiel 79

11-[N-(2-Diethylaminoethyl)-N-ethylamino]-acetyl-6-methyl-2-phenyl-5,6-dihydropyrimido[4.5-b][1.5]benzodiazepinon-(5)

Analog Beispiel 17 erhält man die Titelverbindung aus 11-Chloracetyl-6-methyl-2-phenyl-5,6-dihydropyrimido[4.5-b][1.5]benzodiazepinon-(5) und N,N,N'-Triethylethylendiamin.

Beispiel 80

Tabletten mit 20 mg Wirkstoff

10 kg 6-Methyl-2-phenyl-11-piperidinoacetyl-5,6-dihydropyrimido[4.5-b]-[1.5]benzodiazepinon-(5), 45 kg Milchzucker, 31 kg Maisstärke und 3 kg Polyvinylpyrrolidon werden mit ca. 20 Liter Wasser befeuchtet und durch ein Sieb mit 1,25 mm Maschenweite granuliert. Das Granulat wird im Wirbelschichttrockner bis zu einer relativen Feuchte von 50-60 % getrocknet und dann mit 8 kg Natriumcarboxymethylcellulose, 2 kg Talkum und 1 kg Magnesiumstearat versetzt. Das fertige Granulat wird zu Tabletten à 200 mg und 8 mm Durchmesser verpreßt.

Beispiel 81

Kapseln mit einem Wirkstoffgehalt von 15 mg

150 g 6-Methyl-2-phenyl-11-piperidinoacetyl-5,6-dihydropyrimido[4.5-b]-[1.5]benzodiazepinon-(5), 845 g mikrokirstalline Cellulose und 5 g amorphe Kieselsäure werden feingepulvert, gut vermischt und in Hartgelatinekapseln Größe 4 abgefüllt.

## Pharmakologie

Die ausgezeichnete Magenschutzwirkung der pharmakologisch wirksamen Benzodiazepinone läßt sich am Modell der sogenannten Shay-Ratte nachweisen. Hierbei erweisen sich die erfindungsgemäßen Verbindungen dem bekannten Handelsprodukt Carbenoxolon in der Magenschutzwirkung und der Toxizität eindeutig überlegen.

Die untersuchten Verbindungen sind in der folgenden Tabelle aufgeführt und mit laufenden Nummern versehen worden:

Tabelle I

| lfd. Nr. | Name der Verbindung |
|---|---|
| 1 | Carbenoxolon |
| 2 | 11-[(4-Methyl-1-piperazinyl)acetyl]-5.6-dihydropyrimido-[4.5-b][1.5]benzodiazepinon-(5) |
| 3 | 6-Methyl-11-[(4-methyl-1-piperazinyl)acetyl]-2-phenyl-5.6-dihydropyrimido[4.5-b][1.5]benzodiazepinon-(5) |
| 4 | 6-Methyl-2-phenyl-11-piperidinoacetyl-5.6-dihydropyrimido-[4.5-b][1.5]benzodiazepinon-(5) |
| 5 | 11-[N-(2-Dimethylaminoethyl)-N-methylamino]-acetyl-6-methyl-2-phenyl-5.6-dihydropyrimido[4.5-b][1.5]benzodiazepinon-(5) |

Der Einfluß der erfindungsgemäßen Verbindungen auf die durch Pylorusligatur und 100 mg/kg p.o. Acetylsalicylsäure auslösbare Magengeschwürbildung bei der Ratte und auf die Magensaftsekretion der Ratte wird in der folgenden Tabelle II dargestellt.

Tabelle II

Antiulcerogene Wirkung und Toxizität von Benzodiazepinonen

| lfd. Nr. | Toxizität $LD_{50}$ [mg/kg] i.v. Maus | Magenschutzwirkung $ED_{50}$ [mg/kg] p.o. Ratte | TQ $LD_{50}/ED_{50}$ | Magensekretion*) % Hemmung Ratte Volumen | Salzsäure |
|---|---|---|---|---|---|
| 1 | 290 | ~70 | ~4.1 | 7 | 4 |
| 2 | 180 | 12 | 15 | 10 | 5 |
| 3 | 95 | 9 | 10.5 | 7 | - |
| 4 | 160 | ~8 | ~20 | 32 | 22 |
| 5 | | <10 | | | |

$ED_{50}$ = Dosis, die den Ulcusindex bei der behandelten Gruppe gegenüber der Kontrollgruppe um 50 % mindert

$LD_{50}$ = Dosis, bei der 50 % der Tiere sterben

TQ = Therapeutischer Quotient $LD_{50}/ED_{50}$

*) % Hemmung = Hemmung der Magensekretion ( in %) 4 Stunden nach Applikation der antiulcerogenen $ED_{50}$

Es sei besonders darauf hingewiesen, daß bei Verbindung 1 zwar eine $ED_{50}$ noch bestimmbar ist, die Dosiswirkungskurve dann jedoch sehr stark abflacht, so daß selbst bei 300 mg/kg keine wesentliche Steigerung der antiulcerogenen Wirkung erreichbar ist. Im Gegensatz dazu ist die Wirkung der Verbindungen 2 bis 5 streng dosisabhängig; es lassen sich Hemmeffekte bis 100 % (30 mg/kg, Substanz 5) erreichen.

Die. Prüfung der antiulcerogenen Wirkung erfolgte nach der Methode der sogenannten Shay-Ratte:
Die Ulcusprovokation erfolgt bei 24 Stunden nüchtern gehaltenen Ratten (weiblich, 180 - 200 g, 4 Tiere je Käfig auf hohem Gitterrost) durch Pylorusligatur (unter Diethylethernarkose) und orale Applikation von 100 mg/10 ml/kg Acetylsalicylsäure. Die zu testenden Substanzen werden oral (10 ml/kg) 1 Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) und der Gehalt an Salzsäure bestimmt wurde; mit einem Stereomikroskop werden bei 10-facher Vergößerung Anzahl und Größe (= Durchmesser) vorhandener Ulcera ermittelt. Das Produkt aus Schweregrad (gemäß nachfolgender Punkteskala) und Anzahl der Ulcera dient als individueller Ulcusindex.

Punkteskala:

| | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | >5,5 mm | 6 |

Als Maß für den antiulcerogenen Effekt dient die Minderung des mittleren Ulcus-Index jeder behandelten Gruppe gegenüber dem der Kontrollgruppe (= 100 %). Die $ED_{50}$ bezeichnet die Dosis, die den mittleren Ulcusindex um 50 % mindert.

Bestimmung der Toxizität

Die Toxizitätsuntersuchungen werden an weiblichen NMRI-Mäusen (Körpergewicht 22 - 26 g) durchgeführt. Die Tiere (5 Tiere pro Dosis) erhalten Futter und Wasser ad libitum. Verschiedene Dosen der Substanzen werden intravenös (Injektionsdauer 1 Minute) verabreicht. Die Beobachtungsdauer beträgt 7 Tage. Die $LD_{50}$, d.h. die Dosis, bei der 50 % der Tiere sterben, wird mittels linearer Regression ermittelt.

## Patentansprüche (BE, CH, DE, FR, GB, IT, LU, NL, SE)

1. Benzodiazepinone der allgemeinen Formel I

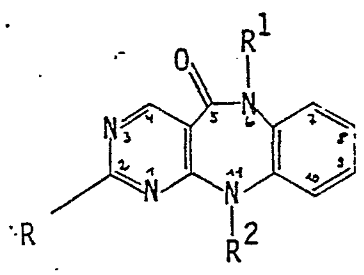

(I),

worin

R ein Wasserstoffatom oder einen Phenylrest,

$R^1$ ein Wasserstoffatom oder einen Alkylrest mit 1 bis 4 Kohlenstoffatomen,

$R^2$ die Gruppe $-CO-C_nH_{2n}-R^3$ oder (wenn R ein Phenylrest ist) ein Wasserstoffatom,

$R^3$ ein Halogenatom oder die Gruppe $-N(R^4)R^5$,

$R^4$ einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihre N-Oxide und ihre Säureadditionssalze mit anorganischen und organischen Säuren.

2. Benzodiazepinone der allgemeinen Formel $I^a$

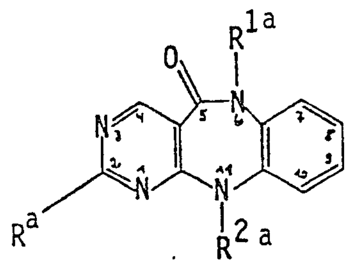

($I^a$),

worin

$R^a$ einen Phenylrest,

$R^{1a}$ ein Wasserstoffatom oder einen Alkylrest mit 1 bis 4 Kohlenstoffatomen,

$R^{2a}$ die Gruppe $-CO-C_{n^a}H_{2n^a}-R^{3a}$,

$R^{3a}$ die Gruppe $-N(R^{4a})R^{5a}$,

$R^{4a}$ einen Alkylrest mit 1 bis 4 Kohlenstoffatomen oder einen Alkenylrest mit 3 bis 5 Kohlenstoffatomen und

$R^{5a}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4a}$ und $R^{5a}$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihre N-Oxide und ihre Säureadditionssalze mit anorganischen und organischen Säuren.

3. Verbindungen nach Anspruch 2, in denen $R^a$, $R^{1a}$, $R^{2a}$ und $R^{3a}$ die dort angegebenen Bedeutungen haben, $R^{4a}$ einen Alkylrest mit 1 bis 4 Kohlenstoffatomen oder einen Alkenylrest mit 3 Kohlenstoffatomen darstellt, $R^{5a}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4a}$ und $R^{5a}$ gemeinsam eine Pyrrolidino-, Piperidino-, Morpholino- oder 4-Methyl- oder 4-Ethyl-1-piperzinylgruppe darstellen, und $n^a$ die Bedeutung 1 hat, und ihre Säureadditionssalze.

4. Verbindungen nach Anspruch 2, in denen $R^a$, $R^{2a}$ und $R^{3a}$ die dort angegebenen Bedeutungen haben, $R^{1a}$ einen Methyl-, n-Propyl-, Isopropyl-, n-Butyl- oder sec.-Butylrest darstellt, $R^{4a}$ einen Methylrest darstellt, $R^{5a}$ einen Methyl- oder Ethylrest darstellt, der endständig durch eine Dimethyl- oder Diethylaminogruppe substituiert ist, oder $R^{4a}$ und $R^{5a}$ gemeinsam eine Pyrrolidino-, Piperidino- oder 4-Methyl-1-piperazinylgruppe darstellen, und $n^a$ die Bedeutung 1 hat, und ihre pharmakologisch verträglichen Säureadditionssalze.

5. Benzodiazepinone der allgemeinen Formel $I^b$

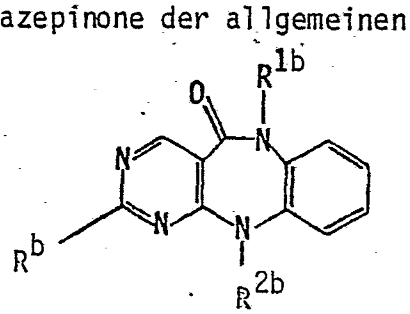

$(I^b)$,

worin

$R^b$ einen Phenylrest,

$R^{1b}$ ein Wasserstoffatom oder einen Alkylrest mit 1 bis 4 Kohlenstoffatomen,

$R^{2b}$ ein Wasserstoffatom oder die Gruppe $-CO-C_{n^b}H_{2n^b}-R^{3b}$,

$R^{3b}$ ein Halogenatom und

$n^b$ 1 oder 2 darstellt,

sowie ihre N-Oxide und ihre Säureadditionssalze mit anorganischen und organischen Säuren.

6. Verbindungen nach Anspruch 5, in denen $R^b$ und $R^{2b}$ die dort angegebenen Bedeutungen haben, $R^{1b}$ einen Methyl-, n-Propyl-, Isopropyl-, n-Butyl- oder sec.-Butylrest darstellt, $R^{3b}$ Chlor oder Brom bedeutet und $n^b$ 1 darstellt und ihre Säureadditionssalze.

7. Benzodiazepinone der allgemeinen Formel $I^c$

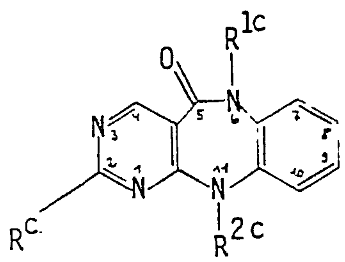

$(I^c)$,

worin

$R^c$ ein Wasserstoffatom,

$R^{1c}$ ein Wasserstoffatom oder einen Alkylrest mit 1 bis 4 Kohlenstoffatomen,

$R^{2c}$ die Gruppe $-CO-C_{n^c}H_{2n^c}-R^{3c}$,

$R^{3c}$ die Gruppe $-N(R^{4c})R^{5c}$,

$R^{4c}$ einen Alkylrest mit 1 bis 4 Kohlenstoffatomen oder einen Alkenylrest mit 3 bis 5 Kohlenstoffatomen und

$R^{5c}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4c}$ und $R^{5c}$ gemeinsam unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

$n^c$ 1 oder 2 darstellt,

sowie ihre N-Oxide und ihre Säureadditionssalze mit anorganischen und organischen Säuren.

8. Verbindungen nach Anspruch 7, in denen $R^c$, $R^{1c}$, $R^{2c}$ und $R^{3c}$ die dort angegebenen Bedeutungen haben, $R^{4c}$ einen Alkylrest mit 1 bis 4 Kohlenstoffatomen oder einen Alkenylrest mit 3 Kohlenstoffatomen darstellt, $R^{5c}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4c}$ und $R^{5c}$ gemeinsam eine Pyrrolidino-, Piperidino-, Morpholino- oder 4-Methyl- oder 4-Ethyl-1-piperazinylgruppe darstellen, und $n^c$ die Bedeutung 1 hat, und ihre Säureadditionssalze.

9. Verbindungen nach Anspruch 7, in denen $R^c$, $R^{2c}$ und $R^{3c}$ die dort angegebenen Bedeutungen haben, $R^{1c}$ ein Wasserstoffatom oder eine Methyl- oder n-Butylgruppe darstellt, $R^{4c}$ einen Methylrest darstellt, $R^{5c}$ einen Methyl- oder Ethylrest darstellt, der endständig durch eine Dimethyl- oder Diethylaminogruppe substituiert ist, oder $R^{4c}$ und $R^{5c}$ gemeinsam eine Pyrrolidino-, Piperidino-, Morpholino- oder 4-Methyl-1-piperazinylgruppe darstellen, und $n^c$ die Bedeutung 1 hat, und ihre pharmakologisch verträglichen Säureadditionssalze.

10. Benzodiazepinone der allgemeinen Formel $I^d$

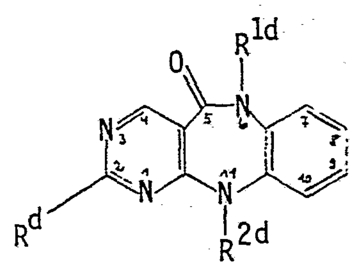

($I^d$),

worin

$R^d$ ein Wasserstoffatom,

$R^{1d}$ ein Wasserstoffatom oder einen Alkylrest mit 1 bis 4 Kohlenstoffatomen,

$R^{2d}$ die Gruppe $-CO-C_{n^d}H_{2n^d}-R^{3d}$,

$R^{3d}$ ein Halogenatom und

$n^d$ 1 oder 2 darstellt,

sowie ihre N-Oxide und ihre Säureadditionssalze mit anorganischen und organischen Säuren.

11. Verbindungen nach Anspruch 10, in denen $R^d$ und $R^{2d}$ die dort angegebenen Bedeutungen haben, $R^{1d}$ ein Wasserstoffatom oder eine Methyl- oder n-Butylgruppe darstellt, $R^{3d}$ ein Chlor- oder Bromatom bedeutet und $n^d$ 1 darstellt, und ihre Säureadditionssalze.

12. Arzneimittel, die ein oder mehrere Benzodiazepinone der allgemeinen Formel I*

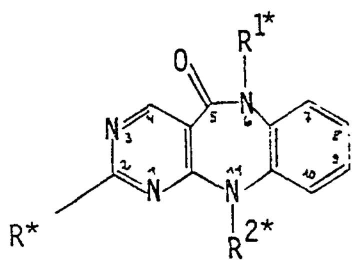

(I*),

worin

R* ein Wasserstoffatom oder einen Phenylrest,

$R^{1*}$ ein Wasserstoffatom oder einen Alkylrest mit 1 bis 4 Kohlenstoffatomen,

$R^{2*}$ die Gruppe $-CO-C_{n^*}H_{2n^*}-R^{3*}$,

$R^{3*}$ die Gruppe $-N(R^{4*})R^{5*}$,

$R^{4*}$ einen Alkylrest mit 1 bis 4 Kohlenstoffatomen oder einen Alkenylrest mit 3 bis 5 Kohlentoffatomen und

$R^{5*}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^{4*}$ und $R^{5*}$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n* 1 oder 2 darstellt,

und/oder ihre pharmakologisch verträglichen N-Oxide und/oder ihre pharmakologisch verträglichen Säureadditionssalze mit anorganischen und organischen Säuren enthalten.

13. Verfahren zur Herstellung von Benzodiazepinonen der allgemeinen Formel I,

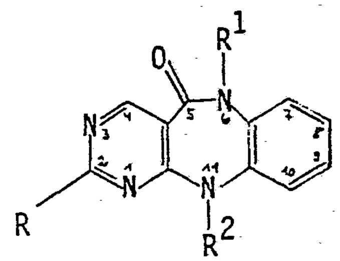

(I),

worin

R ein Wasserstoffatom oder einen Phenylrest,

$R^1$ ein Wasserstoffatom oder einen Alkylrest mit 1 bis 4 Kohlenstoffatomen,

$R^2$ die Gruppe $-CO-C_nH_{2n}-R^3$,

$R^3$ ein Halogenatom oder die Gruppe $-N(R^4)R^5$,

$R^4$ einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihrer N-Oxide und ihrer Säureadditionssalze mit anorganischen und organischen Säuren, dadurch gekennzeichnet, daß man Benzodiazepinone der allgemeinen Formel II

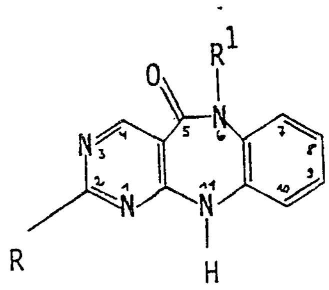

(II),

worin R und $R^1$ die oben angegebenen Bedeutungen haben, mit Verbindungen der allgemeinen Formel III

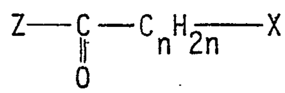

(III),

worin n die oben angegebene Bedeutung hat, X ein Halogenatom (Chlor, Brom, Iod) und Z eine Fluchtgruppe darstellt, umsetzt und gegebenenfalls anschließend mit Aminen der allgemeinen Formel IV

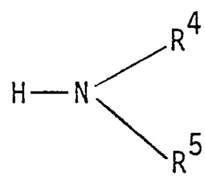

(IV),

worin $R^4$ und $R^5$ die oben angegebenen Bedeutungen haben, reagieren läßt und erhaltene Produkte gewünschtenfalls in die N-Oxide und/oder in die Säureadditionssalze überführt.

## Patentansprüche (AT)

1. Verfahren zur Herstellung von Benzodiazepinonen der allgemeinen Formel I,

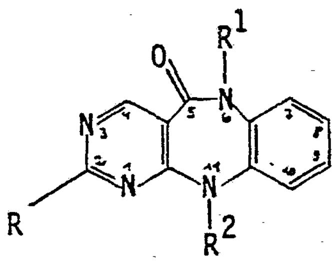

(I),

worin

R ein Wasserstoffatom oder einen Phenylrest,

$R^1$ ein Wasserstoffatom oder einen Alkylrest mit 1 bis 4 Kohlenstoffatomen,

$R^2$ die Gruppe $-CO-C_nH_{2n}-R^3$,

$R^3$ ein Halogenatom oder die Gruppe $-N(R^4)R^5$,

$R^4$ einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihrer N-Oxide und ihrer Säureadditionssalze mit anorganischen und organischen Säuren, dadurch gekennzeichnet, daß man Benzodiazepinone der allgemeinen Formel II

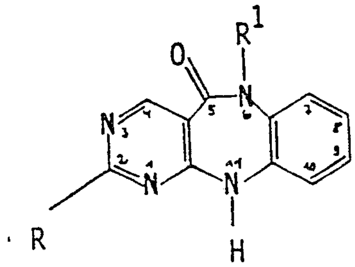

(II),

worin R und $R^1$ die oben angegebenen Bedeutungen haben, mit Verbindungen der allgemeinen Formel III

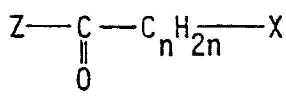

(III),

worin n die oben angegebene Bedeutung hat, X ein Halogenatom (Chlor, Brom, Iod) und Z eine Fluchtgruppe darstellt, umsetzt und gegebenenfalls anschließend mit Aminen der allgemeinen Formel IV

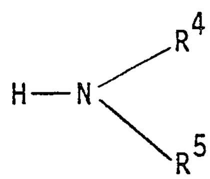

(IV),

worin $R^4$ und $R^5$ die oben angegebenen Bedeutungen haben, reagieren läßt und erhaltene Produkte gewünschtenfalls in die N-Oxide und/oder in die Säureadditionssalze überführt.

2. Verfahren zur Herstellung von Benzodiazepinonen der allgemeinen Formel $I^a$

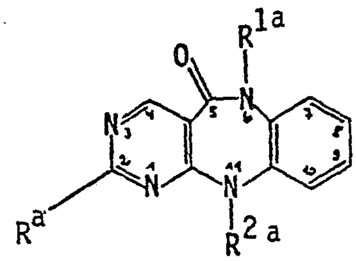

($I^a$),

worin

$R^a$ einen Phenylrest,

$R^{1a}$ ein Wasserstoffatom oder einen Alkylrest mit 1 bis 4 Kohlenstoffatomen,

$R^{2a}$ die Gruppe $-CO-C_{n^a}H_{2n^a}-R^{3a}$,

$R^{3a}$ die Gruppe $-N(R^{4a})R^{5a}$,

$R^{4a}$ einen Alkylrest mit 1 bis 4 Kohlenstoffatomen oder einen Alkenylrest mit 3 bis 5 Kohlenstoffatomen und

$R^{5a}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4a}$ und $R^{5a}$ gemeinsam und unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

n 1 oder 2 darstellt,

sowie ihrer N-Oxide und ihrer Säureadditionssalze mit anorganischen und organischen Säuren, dadurch gekennzeichnet, daß man Benzodiazepinone der allgemeinen Formel $II^a$

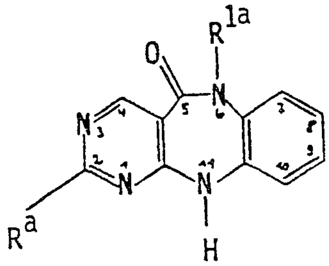

($II^a$),

worin $R^a$ und $R^{1a}$ die oben angegebenen Bedeutungen haben, mit Verbindungen der allgemeinen Formel $III^a$

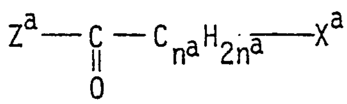

($III^a$),

worin $n^a$ die oben angegebene Bedeutung hat, $X^a$ ein Halogenatom (Chlor, Brom, Iod) und $Z^a$ eine Fluchtgruppe darstellt, umsetzt und anschließend mit Aminen der allgemeinen Formel $IV^a$

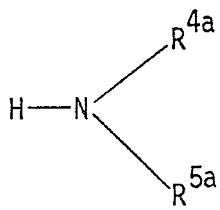

($IV^a$),

worin $R^{4a}$ und $R^{5a}$ die oben angegebenen Bedeutungen haben, reagieren läßt und erhaltene Produkte gewünschtenfalls in die N-Oxide und/ oder in die Säureadditionssalze überführt.

3. Verfahren nach Anspruch 2, dadurch gekennzeichnet, daß man Verbindungen $I^a$, in denen $R^a$, $R^{1a}$, $R^{2a}$ und $R^{3a}$ die dort angegebenen Bedeutungen haben, $R^{4a}$ einen Alkylrest mit 1 bis 4 Kohlenstoffatomen oder einen Alkenylrest mit 3 Kohlenstoffatomen darstellt, $R^{5a}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4a}$ und $R^{5a}$ gemeinsam eine Pyrrolidino-, Piperidino-, Morpholino- oder 4-Methyl- oder 4-Ethyl-1-piperazinylgruppe darstellen, und $n^a$ die Bedeutung 1 hat, und ihre Säure -

additionssalze herstellt, indem man entsprechend substituierte Verbindungen $II^a$, $III^a$ und $IV^a$ miteinander umsetzt, wobei $Z^a$ eine Alkoxy-, Hydroxy- oder Halogenfluchtgruppe darstellt und $X^a$ ein Chlor- oder Bromatom ist.

4. Verfahren nach Anspruch 2, dadurch gekennzeichnet, daß man Verbindungen $I^a$, in denen $R^a$, $R^{2a}$ und $R^{3a}$ die dort angegebenen Bedeutungen haben, $R^{1a}$ einen Methyl-, n-Propyl-, Isopropyl-, n-Butyl- oder sec.-Butylrest darstellt, $R^{4a}$ einen Methylrest darstellt, $R^{5a}$ einen Methyl- oder Ethylrest darstellt, der endständig durch eine Dimethyl- oder Diethylaminogruppe substituiert ist, oder $R^{4a}$ und $R^{5a}$ gemeinsam eine Pyrrolidino-, Piperidino- oder 4-Methyl-1-piperazinylgruppe darstellen, und $n^a$ die Bedeutung 1 hat, und ihre pharmakologisch verträglichen Säureadditionssalze herstellt, indem man entsprechend substituierte Verbindungen $II^a$, $III^a$ und $IV^a$ miteinander umsetzt, wobei $Z^a$ einen Ethoxy-, Methoxy-, Chlor- oder Bromrest darstellt und $X^a$ ein Chlor- oder Bromatom ist.

5. Verfahren zur Herstellung von Benzodiazepinonen der allgemeinen Formel $I^b$

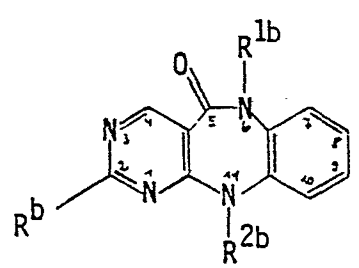

$(I^b)$,

worin
$R^b$ einen Phenylrest,
$R^{1b}$ ein Wasserstoffatom oder einen Alkylrest mit 1 bis 4 Kohlenstoffatomen,
$R^{2b}$ ein Wasserstoffatom oder die Gruppe $-CO-C_{n^b}H_{2n^b}-R^{3b}$,
$R^{3b}$ ein Halogenatom und
$n^b$ 1 oder 2 darstellt,
sowie ihrer N-Oxide und ihrer Säureadditionssalze mit anorganischen

und organischen Säuren, dadurch gekennzeichnet, daß man Benzodiazepinone der allgemeinen Formel $II^b$

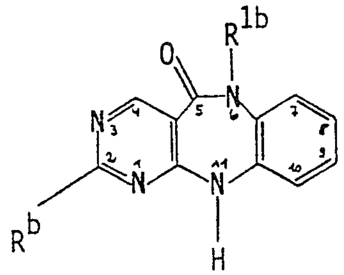

($II^b$),

worin $R^b$ und $R^{1b}$ die oben angegebenen Bedeutungen haben, mit Verbindungen der allgemeinen Formel $III^b$

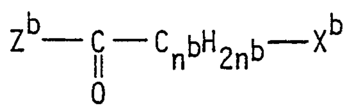

($III^b$),

worin $n^b$ die oben angegebene Bedeutung hat, $X^b$ ein Halogenatom (Chlor, Brom, Iod) und $Z^b$ eine Fluchtgruppe darstellt, umsetzt und gegebenenfalls anschließend in die N-Oxide und/oder in die Säureadditionssalze überführt.

6. Verfahren nach Anspruch 5, dadurch gekennzeichnet, daß man Verbindungen $I^b$, in denen $R^b$ und $R^{2b}$ die oben angegebenen Bedeutungen haben, $R^{1b}$ einen Methyl-, n-Propyl-, Isopropyl-, n-Butyl- oder sec.-Butylrest darstellt, $R^{3b}$ Chlor oder Brom bedeutet und $n^b$ 1 darstellt und ihre Säureadditionssalze herstellt, indem man entsprechend substituierte Verbindungen $II^b$ und $III^b$ miteinander umsetzt, wobei $Z^b$ einen Ethoxy-, Methoxy-, Chlor- oder Bromrest darstellt und $X^b$ ein Chlor- oder Bromatom ist.

7. Verfahren zur Herstellung von Benzodiazepinonen der allgemeinen Formel $I^c$

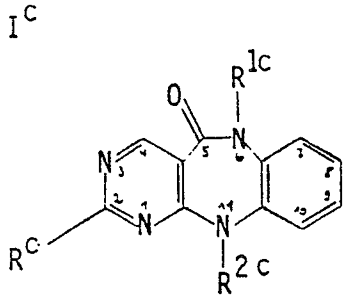

($I^c$),

worin

$R^c$ ein Wasserstoffatom,

$R^{1c}$ ein Wasserstoffatom oder einen Alkylrest mit 1 bis 4 Kohlenstoffatomen,

$R^{2c}$ die Gruppe $-CO-C_{n^c}H_{2n^c}-R^{3c}$,

$R^{3c}$ die Gruppe $-N(R^{4c})R^{5c}$,

$R^{4c}$ einen Alkylrest mit 1 bis 4 Kohlenstoffatomen oder einen Alkenylrest mit 3 bis 5 Kohlenstoffatomen und

$R^{5c}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4c}$ und $R^{5c}$ gemeinsam unter Einschluß des Stickstoffatoms an das sie gebunden sind einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten und

$n^c$ 1 oder 2 darstellt,

sowie ihrer N-Oxide und ihrer Säureadditionssalze mit anorganischen und organischen Säuren, dadurch gekennzeichnet, daß man Benzodiazepinone der allgemeinen Formel $II^c$

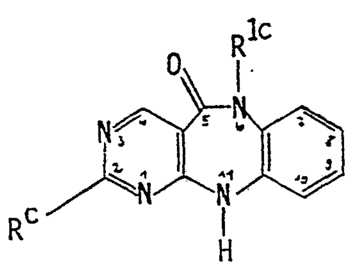

($II^c$),

worin $R^c$ und $R^{1c}$ die oben angegebenen Bedeutungen haben, mit Verbindungen der allgemeinen Formel $III^c$

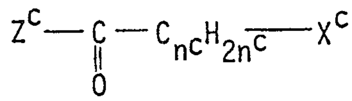

($III^c$),

worin $n^c$ die oben angegebene Bedeutung hat, $X^c$ ein Halogenatom (Chlor, Brom, Iod) und $Z^c$ eine Fluchtgruppe darstellt, umsetzt und anschließend mit Aminen der allgemeinen Formel $IV^c$

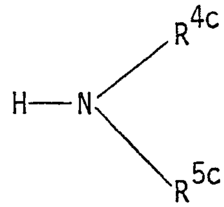

($IV^c$),

worin $R^{4c}$ und $R^{5c}$ die oben angegebenen Bedeutungen haben, reagieren läßt und erhaltene Produkte gewünschtenfalls in die N-Oxide und/ oder in die Säureadditionssalze überführt.

8. Verfahren nach Anspruch 7, dadurch gekennzeichnet, daß man Verbindungen $I^c$, in denen $R^c$, $R^{1c}$, $R^{2c}$ und $R^{3c}$ die dort angegebenen Bedeutungen haben, $R^{4c}$ einen Alkylrest mit 1 bis 4 Kohlenstoffatomen oder einen Alkenylrest mit 3 Kohlenstoffatomen darstellt, $R^{5c}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4c}$ und $R^{5c}$ gemeinsam eine Pyrrolidino-, Piperidino-, Morpholino- oder 4-Methyl- oder 4-Ethyl-1-piperazinylgruppe darstellen, und $n^c$ die Bedeutung 1 hat, und ihre Säureadditionssalze herstellt, indem man entsprechend substituierte Verbindungen $II^c$, $III^c$ und $IV^c$ miteinander umsetzt, wobei $Z^c$ eine Alkoxy-, Hydroxy- oder Halogenfluchtgruppe darstellt, und $X^c$ ein Chlor- oder Bromatom ist.

9. Verfahren nach Anspruch 7, dadurch gekennzeichnet, daß man Verbindungen $I^c$, in denen $R^c$, $R^{2c}$ und $R^{3c}$ die dort angegebenen Bedeutungen haben, $R^{1c}$ ein Wasserstoffatom oder eine Methyl- oder n-Butylgruppe darstellt, $R^{4c}$ einen Methylrest darstellt, $R^{5c}$ einen Methyl- oder Ethylrest darstellt, der endständig durch eine Dimethyl- oder Diethylaminogruppe substituiert ist, oder $R^{4c}$ und $R^{5c}$

gemeinsam eine Pyrrolidino-, Piperidino-, Morpholino- oder 4-Methyl-1-piperazinylgruppe darstellen, und $n^c$ die Bedeutung 1 hat, und ihre pharmakologisch verträglichen Säureadditionssalze herstellt, indem man entsprechend substituierte Verbindungen $II^c$, $III^c$ und $IV^c$ miteinander umsetzt, wobei $Z^c$ einen Ethoxy-, Methoxy-, Chlor- oder Bromrest darstellt und $X^c$ ein Chlor- oder Bromatom ist.

10. Verfahren zur Herstellung von Benzodiazepinonen der allgemeinen Formel $I^d$

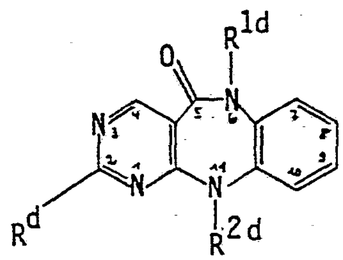

($I^d$),

worin

$R^d$ ein Wasserstoffatom,

$R^{1d}$ ein Wasserstoffatom oder einen Alkylrest mit 1 bis 4 Kohlenstoffatomen,

$R^{2d}$ die Gruppe $-CO-C_{n^d}H_{2n^d}-R^{3d}$,

$R^{3d}$ ein Halogenatom und

$n^d$ 1 oder 2 darstellt,

sowie ihrer N-Oxide und ihrer Säureadditionssalze mit anorganischen und organischen Säuren, dadurch gekennzeichnet, daß man Benzodiazepinone der allgemeinen Formel $II^d$

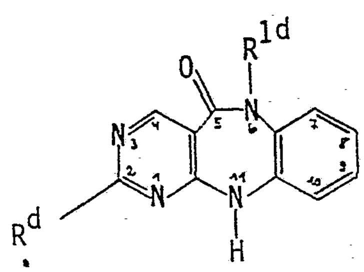

($II^d$),

worin $R^d$ und $R^{1d}$ die oben angegebenen Bedeutungen haben, mit Verbindungen der allgemeinen Formel $III^d$

$$Z^d - \underset{\underset{O}{\|}}{C} - C_{n^d}H_{2n^d} - X^d \qquad (III^d),$$

worin $n^d$ die oben angegebene Bedeutung hat, $X^d$ ein Halogenatom (Chlor, Brom, Iod) und $Z^d$ eine Fluchtgruppe darstellt, umsetzt und gegebenenfalls anschließend in die N-Oxide und/oder in die Säureadditionssalze überführt.

11. Verfahren nach Anspruch 7, dadurch gekennzeichnet, daß man Verbindungen $I^d$, in denen $R^d$ und $R^{2d}$ die oben angegebenen Bedeutungen haben, $R^{1d}$ ein Wasserstoffatom oder eine Methyl- oder n-Butylgruppe darstellt, $R^{3d}$ ein Chlor- oder Bromatom bedeutet und $n^d$ 1 darstellt, und ihre Säureadditionssalze herstellt, indem man entsprechend substituierte Verbindungen $II^d$ und $III^d$ miteinander umsetzt, wobei $Z^d$ einen Ethoxy-, Methoxy-, Chlor- oder Bromrest darstellt und $X^d$ ein Chlor- oder Bromatom ist.

0024582

Europäisches Patentamt

**EUROPÄISCHER RECHERCHENBERICHT**

Nummer der Anmeldung

EP 80104589.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich der maßgeblichen Teile | betrifft Anspruch | |
| | DE - A1 - 2 424 811 (THOMAE)<br>+ Patentansprüche +<br>--<br>DE - A1 - 2 707 270 (HOECHST)<br>+ Patentansprüche +<br>--<br>US - A - 3 872 122 (JUBY, HUDYMA)<br>+ Gesamt +<br>---- | 1,12,13<br><br><br>1,12,13<br><br><br>1,12,13 | C 07 D 487/04<br>A 61 K 31/55//<br>(C 07 D 487/04<br>C 07 D 239/00<br>C 07 D 243/00) |
| | | | RECHERCHIERTE SACHGEBIETE (Int. Cl.³) |
| | | | C 07 D 471/00<br>C 07 D 487/00<br>A 61 K 31/00 |
| | | | KATEGORIE DER GENANNTEN DOKUMENTE |
| | | | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 20-10-1980 | LUX |